# EUROPEAN PATENT APPLICATION

(11) **EP 3 409 322 A1**
(43) Date of publication of application: **05.12.2018**
(21) Application number: 17173980.8
(22) Date of filing: 01.06.2017
(51) Int. Cl.: A61P 35/00, C07K 16/28, A61K 39/395, A61K 31/573, C07K 16/30, A61K 39/00

(54) **TREATMENT METHOD**

(71) Applicant: F. Hoffmann-La Roche AG, 4070 Basel (CH)
(72) Inventor: The designation of the inventor has not yet been filed
(74) Representative: Cueni, Leah Noëmi

(57) **Abstract**

The present invention relates to the treatment of cancer, in particular to the prevention of adverse effects caused by the administration of CD3 antibodies in the course of said treatment.

## Description

### Field of the Invention

The present invention relates to the treatment of cancer, in particular to the prevention of adverse effects caused by the administration of CD3 antibodies in the course of said treatment.

### Background

T cell activating antibodies are a novel class of cancer therapeutics, designed to engage cytotoxic T cells against tumor cells. The simultaneous binding of such an antibody to CD3 on T cells and to an antigen expressed on the tumor cells will force a temporary interaction between tumor cell and T cell, causing activation of the T cell and subsequent lysis of the tumor cell.

Through their mechanism of action, T cell activating antibodies can lead to inflammation of tumors, in particular solid tumors, characterized e.g. by the increased presence of lymphocytic infiltrates and chemokines in the tumor. This inflammation reaction, however, can give rise to adverse effects which have to be controlled for the safety of the patient. The nature of these tumor inflammation-related adverse effects is diverse and largely depends on the size and location of tumor. For example, inflammation (associated i.a. with swelling) of a tumor in the lung may give rise to hypoxia, while inflammation of a tumor in the in the peritoneum may lead to enteritis, inflammation of a tumor in the liver to increases in liver enzymes, and inflammation of a tumor in colon to colitis.

To fully exploit the potential of these much needed, highly active cancer therapeutics, it is critical to understand and mitigate adverse effects that may occur. The present invention provides means to effectively control tumor inflammation-related adverse effects, which are associated with the mechanism of action of CD3 (bispecific) antibodies and occur particularly in the treatment of solid tumor cancers with such antibodies.

### Summary of the Invention

The present invention is based, at least in part, on the recognition that CD3 antibodies, in particular bispecific antibodies directed to CD3 and a tumor target, most particular bispecific antibodies directed to CD3 and a solid tumor target, may induce inflammation of tumors and thereby cause adverse effects related to that inflammation (called herein "tumor inflammation-related adverse effects"). The present inventors have further found that these effects may be effectively ameliorated, treated or prevented by administration of a glucocorticoid (GC) in the course of the treatment with the CD3 antibody.

Accordingly, the present invention establishes for the first time that tumor inflammation-related adverse effects may occur in the course of the treatment of a cancer, in particular of a solid tumor cancer, with a CD3 antibody, particularly a bispecific antibody directed to CD3 and a tumor target, and that these adverse effects can be mitigated or even prevented if the administration of the CD3 antibody is preceded, accompanied or followed, in particular followed, by administration of a glucocorticoid.

Thus, in a first aspect, the invention provides a CD3 antibody for use in the treatment of cancer, wherein said treatment comprises administration of a glucocorticoid (GC) for the amelioration, treatment or prophylaxis of tumor inflammation-related adverse events caused by (the administration of) the CD3 antibody.

In a further aspect, the invention provides a method of treating cancer, comprising administration of a CD3 antibody and of a glucocorticoid (GC), wherein the GC is administered for the amelioration, treatment or prophylaxis of tumor inflammation-related adverse events caused by (the administration of) the CD3 antibody.

In still a further aspect, the invention provides the use of a CD3 antibody in the manufacture of a medicament for the treatment of cancer, wherein said treatment comprises administration of a glucocorticoid (GC) for the amelioration, treatment or prophylaxis of tumor inflammation-related adverse events caused by (the administration of) the CD3 antibody.

In some embodiments of the CD3 antibodies, methods or uses described above and herein, the GC is selected from methylprednisolone and prednisone. In some embodiments, the GC is administered at a dose of about 5 to about 60 mg, particularly about 40 mg.

In some embodiments, the GC is administered before, concurrently with and/or after the administration of the CD3 antibody. In particular embodiments, the GC is administered after the administration of the CD3 antibody. In more particular embodiments, the GC is administered between about 1 hour and about 7 days after the administration, particularly the first administration, of the CD3 antibody. In a particular embodiment, the GC is administered (i) at about 3 hours, about 1 day (24 hours), about 2 days (48 hours) and about 3 days (72 hours), or (ii) at about 3 hours, about 1 day (24 hours), about 2 days (48 hours), about 3 days (72 hours), about 4 days (96 hours), about 5 days (120 hours), about 6 days (144 hours) and about 7 days (168 hours) after the (first) administration of the CD3 antibody.

In some embodiments, the cancer is a solid tumor cancer. In particular embodiments, the cancer is a cancer selected from the group consisting of colorectal cancer, lung cancer, pancreatic cancer, breast cancer, and gastric cancer.

In a particular embodiment, the CD3 antibody comprises a heavy chain variable region comprising the heavy chain CDR (HCDR) 1 of SEQ ID NO: 1, the HCDR2 of SEQ ID NO: 2, and the HCDR3 of SEQ ID NO: 3; and a light chain variable region comprising the light chain CDR (LCDR) 1 of SEQ ID NO: 4, the LCDR2 of SEQ ID NO: 5 and the LCDR3 of SEQ ID NO: 6.

In some embodiments, the CD3 antibody is a bispecific antibody that specifically binds to CD3 and to a target cell antigen. In particular embodiments, the CD3 antibody is a bispecific antibody that specifically binds to CD3 and to CEA.

In a particular embodiment, the CD3 antibody is a bispecific antibody comprising
(i) a first antigen binding moiety that specifically binds to CD3 and comprises a heavy chain variable region comprising the heavy chain CDR (HCDR) 1 of SEQ ID NO: 1, the HCDR2 of SEQ ID NO: 2, and the HCDR3 of SEQ ID NO: 3; and a light chain variable region comprising the light chain CDR (LCDR) 1 of SEQ ID NO: 4, the LCDR2 of SEQ ID NO: 5 and the LCDR3 of SEQ ID NO: 6; and
(ii) a second antigen binding moiety that specifically binds to CEA and comprises (i) a heavy chain variable region comprising the heavy chain CDR (HCDR) 1 of SEQ ID NO: 9, the HCDR2 of SEQ ID NO: 10, and the HCDR3 of SEQ ID NO: 11; and a light chain variable region comprising the light chain CDR (LCDR) 1 of SEQ ID NO: 12, the LCDR2 of SEQ ID NO: 13 and the LCDR3 of SEQ ID NO: 14; or (ii) a heavy chain variable region comprising the heavy chain CDR (HCDR) 1 of SEQ ID NO: 17, the HCDR2 of SEQ ID NO: 18, and the HCDR3 of SEQ ID NO: 19; and a light chain variable region comprising the light chain CDR (LCDR) 1 of SEQ ID NO: 20, the LCDR2 of SEQ ID NO: 21 and the LCDR3 of SEQ ID NO: 22.

In one embodiment, the CD3 antibody is CEA TCB.

In some embodiments, the CD3 antibody is administered at a dose of about 60 mg to about 1200 mg. In some embodiments, the CD3 antibody is administered every week / once a week (QW).

In some embodiments, the CD3 antibody is used in combination with a PD-1 axis binding antagonist. In some embodiments, the CD3 antibody is used in combination with atezolizumab. In some embodiments, atezolizumab is administered at a dose of about 1200 mg every three weeks.

### Detailed Description of the Embodiments

Terms are used herein as generally used in the art, unless otherwise defined in the following.

The term "bispecific" means that the antigen binding molecule is able to specifically bind to at least two distinct antigenic determinants. Typically, a bispecific antigen binding molecule comprises two antigen binding sites, each of which is specific for a different antigenic determinant. In certain embodiments the bispecific antigen binding molecule is capable of simultaneously binding two antigenic determinants, particularly two antigenic determinants expressed on two distinct cells.

The term "valent" as used herein denotes the presence of a specified number of antigen binding sites in an antigen binding molecule. As such, the term "monovalent binding to an antigen" denotes the presence of one (and not more than one) antigen binding site specific for the antigen in the antigen binding molecule.

As used herein, the term "antigen binding moiety" refers to a polypeptide molecule that specifically binds to an antigenic determinant. In one embodiment, an antigen binding moiety is able to direct the entity to which it is attached (e.g. a second antigen binding moiety) to a target site, for example to a specific type of tumor cell bearing the antigenic determinant. In another embodiment an antigen binding moiety is able to activate signaling through its target antigen, for example a T cell receptor complex antigen. Antigen binding moieties include antibodies and fragments thereof as further defined herein. Particular antigen binding moieties include an antigen binding domain of an antibody, comprising an antibody heavy chain variable region and an antibody light chain variable region. In certain embodiments, the antigen binding moieties may comprise antibody constant regions as further defined herein and known in the art. Useful heavy chain constant regions include any of the five isotypes: α, δ, ε, γ, or µ. Useful light chain constant regions include any of the two isotypes: κ and λ.

As used herein, the term "antigenic determinant" is synonymous with "antigen" and "epitope", and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM).

By "specific binding" is meant that the binding is selective for the antigen and can be discriminated from unwanted or non-specific interactions. The ability of an antigen binding moiety to bind to a specific antigenic determinant can be measured either through an enzyme-linked immunosorbent assay (ELISA) or other techniques familiar to one of skill in the art, e.g. surface plasmon resonance (SPR) technique (analyzed e.g. on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding moiety to an unrelated protein is less than about 10% of the binding of the antigen binding moiety to the antigen as measured, e.g., by SPR. In certain embodiments, an antigen binding moiety that binds to the antigen, or an antigen binding molecule comprising that antigen binding moiety, has a dissociation constant (K_{D}) of ≤ 1 µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

"Affinity" refers to the strength of the sum total of non-covalent interactions between a single binding site of a molecule (e.g., a receptor) and its binding partner (e.g., a ligand). Unless indicated otherwise, as used herein, "binding affinity" refers to intrinsic binding affinity which reflects a 1:1 interaction between members of a binding pair (e.g., an antigen binding moiety and an antigen, or a receptor and its ligand). The affinity of a molecule X for its partner Y can generally be represented by the dissociation constant (K_{D}), which is the ratio of dissociation and association rate constants (k_{off} and kₒₙ, respectively). Thus, equivalent affinities may comprise different rate constants, as long as the ratio of the rate constants remains the same. Affinity can be measured by well established methods known in the art, including those described herein. A particular method for measuring affinity is Surface Plasmon Resonance (SPR).

"Reduced binding", for example reduced binding to an Fc receptor, refers to a decrease in affinity for the respective interaction, as measured for example by SPR. For clarity, the term includes also reduction of the affinity to zero (or below the detection limit of the analytic method), i.e. complete abolishment of the interaction. Conversely, "increased binding" refers to an increase in binding affinity for the respective interaction.

"CD3" refers to any native CD3 from any vertebrate source, including mammals such as primates (e.g. humans), non-human primates (e.g. cynomolgus monkeys) and rodents (e.g. mice and rats), unless otherwise indicated. The term encompasses "full-length," unprocessed CD3 as well as any form of CD3 that results from processing in the cell. The term also encompasses naturally occurring variants of CD3, e.g., splice variants or allelic variants. In one embodiment, CD3 is human CD3, particularly the epsilon subunit of human CD3 (CD3ε). The amino acid sequence of human CD3ε is shown in UniProt (www.uniprot.org) accession no. P07766 (version 144), or NCBI (www.ncbi.nlm.nih.gov/) RefSeq NP_000724.1. See also SEQ ID NO: 34. The amino acid sequence of cynomolgus [Macaca fascicularis] CD3ε is shown in NCBI GenBank no. BAB71849.1. See also SEQ ID NO: 35.

"T cell activation" as used herein refers to one or more cellular response of a T lymphocyte, particularly a cytotoxic T lymphocyte, selected from: proliferation, differentiation, cytokine secretion, cytotoxic effector molecule release, cytotoxic activity, and expression of activation markers. Suitable assays to measure T cell activation are known in the art and described herein.

A "target cell antigen" as used herein refers to an antigenic determinant presented on the surface of a target cell, for example a cell in a tumor such as a cancer cell or a cell of the tumor stroma. In a particular embodiment, the target cell antigen is CEA, particularly human CEA.

"Carcinoembryonic antigen" or "CEA" (also known as Carcinoembryonic antigen-related cell adhesion molecule 5 (CEACAM5)) refers to any native CEA from any vertebrate source, including mammals such as primates (e.g. humans), non-human primates (e.g. cynomolgus monkeys) and rodents (e.g. mice and rats), unless otherwise indicated. The term encompasses "full-length," unprocessed CEA as well as any form of CEA that results from processing in the cell. The term also encompasses naturally occurring variants of CEA, e.g., splice variants or allelic variants. In one embodiment, CEA is human CEA. The amino acid sequence of human CEA is shown in UniProt (www.uniprot.org) accession no. P06731, or NCBI (www.ncbi.nlm.nih.gov/) RefSeq NP_004354.2.

As used herein, the terms "first", "second" or "third" with respect to Fab molecules etc., are used for convenience of distinguishing when there is more than one of each type of moiety. Use of these terms is not intended to confer a specific order or orientation of the bispecific antigen binding molecule unless explicitly so stated.

By "fused" is meant that the components (e.g. a Fab molecule and an Fc domain subunit) are linked by peptide bonds, either directly or via one or more peptide linkers.

A "Fab molecule" refers to a protein consisting of the VH and CH1 domain of the heavy chain (the "Fab heavy chain") and the VL and CL domain of the light chain (the "Fab light chain") of an immunoglobulin.

By a "crossover" Fab molecule (also termed "Crossfab") is meant a Fab molecule wherein the variable domains or the constant domains of the Fab heavy and light chain are exchanged (i.e. replaced by each other), i.e. the crossover Fab molecule comprises a peptide chain composed of the light chain variable domain VL and the heavy chain constant domain 1 CH1 (VL-CH1, in N-to C-terminal direction), and a peptide chain composed of the heavy chain variable domain VH and the light chain constant domain CL (VH-CL, in N- to C-terminal direction). For clarity, in a crossover Fab molecule wherein the variable domains of the Fab light chain and the Fab heavy chain are exchanged, the peptide chain comprising the heavy chain constant domain 1 CH1 is referred to herein as the "heavy chain" of the (crossover) Fab molecule. Conversely, in a crossover Fab molecule wherein the constant domains of the Fab light chain and the Fab heavy chain are exchanged, the peptide chain comprising the heavy chain variable domain VH is referred to herein as the "heavy chain" of the (crossover) Fab molecule.

In contrast thereto, by a "conventional" Fab molecule is meant a Fab molecule in its natural format, i.e. comprising a heavy chain composed of the heavy chain variable and constant domains (VH-CH1, in N- to C-terminal direction), and a light chain composed of the light chain variable and constant domains (VL-CL, in N- to C-terminal direction).

The term "immunoglobulin molecule" refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable domain (VH), also called a variable heavy domain or a heavy chain variable region, followed by three constant domains (CH1, CH2, and CH3), also called a heavy chain constant region. Similarly, from N- to C-terminus, each light chain has a variable domain (VL), also called a variable light domain or a light chain variable region, followed by a constant light (CL) domain, also called a light chain constant region. The heavy chain of an immunoglobulin may be assigned to one of five types, called α (IgA), δ (IgD), ε (IgE), γ (IgG), or µ (IgM), some of which may be further divided into subtypes, e.g. γ₁ (IgG₁), γ₂ (IgG₂), γ₃ (IgG₃), γ₄ (IgG₄), α₁ (IgA₁) and α₂ (IgA₂). The light chain of an immunoglobulin may be assigned to one of two types, called kappa (κ) and lambda (λ), based on the amino acid sequence of its constant domain. An immunoglobulin essentially consists of two Fab molecules and an Fc domain, linked via the immunoglobulin hinge region.

The term "antibody" herein is used in the broadest sense and encompasses various antibody structures, including but not limited to monoclonal antibodies, polyclonal antibodies, multispecific antibodies (e.g. bispecific antibodies), and antibody fragments so long as they exhibit the desired antigen-binding activity.

The terms "full length antibody," "intact antibody," and "whole antibody" are used herein interchangeably to refer to an antibody having a structure substantially similar to a native antibody structure.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂, diabodies, linear antibodies, single-chain antibody molecules (e.g. scFv), and single-domain antibodies. For a review of certain antibody fragments, see Hudson et al., Nat Med 9, 129-134 (2003). For a review of scFv fragments, see e.g. Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046. Diabodies are antibody fragments with two antigen-binding sites that may be bivalent or bispecific. See, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

The term "antigen binding domain" refers to the part of an antibody that comprises the area which specifically binds to and is complementary to part or all of an antigen. An antigen binding domain may be provided by, for example, one or more antibody variable domains (also called antibody variable regions). Particularly, an antigen binding domain comprises an antibody light chain variable domain (VL) and an antibody heavy chain variable domain (VH).

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen-binding specificity. As used herein in connection with variable region sequences, "Kabat numbering" refers to the numbering system set forth by Kabat et al., Sequences of Proteins of Immunological Interest, 5th Ed. Public Health Service, National Institutes of Health, Bethesda, MD (1991).

As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991), referred to as "numbering according to Kabat" or "Kabat numbering" herein. Specifically the Kabat numbering system (see pages 647-660 of Kabat, et al., Sequences of Proteins of Immunological Interest, 5th ed., Public Health Service, National Institutes of Health, Bethesda, MD (1991)) is used for the light chain constant domain CL of kappa and lambda isotype and the Kabat EU index numbering system (see pages 661-723) is used for the heavy chain constant domains (CH1, Hinge, CH2 and CH3), which is herein further clarified by referring to "numbering according to Kabat EU index" in this case.

The term "hypervariable region" or "HVR", as used herein, refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs; three in the VH (H1, H2, H3), and three in the VL (L1, L2, L3). Exemplary HVRs herein include:
(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following order in VH (or VL): FR1-H1(L1)-FR2-H2(L2)-FR3-H3(L3)-FR4.

The "class" of an antibody or immunoglobulin refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

The term "Fc domain" or "Fc region" herein is used to define a C-terminal region of an immunoglobulin heavy chain that contains at least a portion of the constant region. The term includes native sequence Fc regions and variant Fc regions. Although the boundaries of the Fc region of an IgG heavy chain might vary slightly, the human IgG heavy chain Fc region is usually defined to extend from Cys226, or from Pro230, to the carboxyl-terminus of the heavy chain. However, antibodies produced by host cells may undergo post-translational cleavage of one or more, particularly one or two, amino acids from the C-terminus of the heavy chain. Therefore an antibody produced by a host cell by expression of a specific nucleic acid molecule encoding a full-length heavy chain may include the full-length heavy chain, or it may include a cleaved variant of the full-length heavy chain. This may be the case where the final two C-terminal amino acids of the heavy chain are glycine (G446) and lysine (K447, numbering according to Kabat EU index). Therefore, the C-terminal lysine (Lys447), or the C-terminal glycine (Gly446) and lysine (K447), of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991 (see also above). A "subunit" of an Fc domain as used herein refers to one of the two polypeptides forming the dimeric Fc domain, i.e. a polypeptide comprising C-terminal constant regions of an immunoglobulin heavy chain, capable of stable self-association. For example, a subunit of an IgG Fc domain comprises an IgG CH2 and an IgG CH3 constant domain.

A "modification promoting the association of the first and the second subunit of the Fc domain" is a manipulation of the peptide backbone or the post-translational modifications of an Fc domain subunit that reduces or prevents the association of a polypeptide comprising the Fc domain subunit with an identical polypeptide to form a homodimer. A modification promoting association as used herein particularly includes separate modifications made to each of the two Fc domain subunits desired to associate (i.e. the first and the second subunit of the Fc domain), wherein the modifications are complementary to each other so as to promote association of the two Fc domain subunits. For example, a modification promoting association may alter the structure or charge of one or both of the Fc domain subunits so as to make their association sterically or electrostatically favorable, respectively. Thus, (hetero)dimerization occurs between a polypeptide comprising the first Fc domain subunit and a polypeptide comprising the second Fc domain subunit, which might be non-identical in the sense that further components fused to each of the subunits (e.g. antigen binding moieties) are not the same. In some embodiments the modification promoting association comprises an amino acid mutation in the Fc domain, specifically an amino acid substitution. In a particular embodiment, the modification promoting association comprises a separate amino acid mutation, specifically an amino acid substitution, in each of the two subunits of the Fc domain.

The term "effector functions" refers to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, Clustal W, Megalign (DNASTAR) software or the FASTA program package. Those skilled in the art can determine appropriate parameters for aligning sequences, including any algorithms needed to achieve maximal alignment over the full length of the sequences being compared. For purposes herein, however, % amino acid sequence identity values are generated using the ggsearch program of the FASTA package version 36.3.8c or later with a BLOSUM50 comparison matrix. The FASTA program package was authored by W. R. Pearson and D. J. Lipman (1988), "Improved Tools for Biological Sequence Analysis", PNAS 85:2444-2448; W. R. Pearson (1996) "Effective protein sequence comparison" Meth. Enzymol. 266:227- 258; and Pearson et. al. (1997) Genomics 46:24-36, and is publicly available from http://fasta.bioch.virginia.edu/fasta_www2/fasta_down.shtml. Alternatively, a public server accessible at http://fasta.bioch.virginia.edu/fasta_www2/index.cgi can be used to compare the sequences, using the ggsearch (global protein:protein) program and default options (BLOSUM50; open: -10; ext: -2; Ktup = 2) to ensure a global, rather than local, alignment is performed. Percent amino acid identity is given in the output alignment header.

An "activating Fc receptor" is an Fc receptor that following engagement by an Fc domain of an antibody elicits signaling events that stimulate the receptor-bearing cell to perform effector functions. Human activating Fc receptors include FcγRIIIa (CD16a), FcγRI (CD64), FcγRIIa (CD32), and FcαRI (CD89).

An "adverse effect", which is sometimes also denoted as "side effect" or "adverse event" (in clinical studies) is a harmful and undesired effect resulting from medication in the treatment of a patient with a CD3 antibody.

A "tumor inflammation-related adverse effect" denotes conditions of a cancer patient that are related to inflammation of his tumor(s) caused by the administration of a CD3 antibody (particularly a bispecific antibody directed to CD3 and a tumor target). The nature of tumor inflammation-related adverse effects is diverse and to a large extent depends on the size and location of tumor. Tumor inflammation-related adverse effects may include, for example, hypoxia (especially if the tumor is located in the lung), enteritis (especially if the tumor is located in the peritoneum), increase in liver enzymes (especially if the tumor is located in the liver) and colitis (especially if the tumor is located in the colon).

Tumor inflammation-related adverse effects generally arise between about 24 hours and about 7 days, mostly between about 24 and about 72 hours, after administration of the CD3 antibody, when tumor inflammation occurs. They occur mainly after the first administration of the CD3 antibody, with a smaller likelihood of (re-)occurring after subsequent administrations. Tumor inflammation can be detected by techniques known in the art, such as, for example, computer tomography (CT) or biopsies. Signs of tumor inflammation include enlargement of tumor lesions and perilesional edema (as detected e.g. by CT), or infiltration of T cells, particularly CD8+ T cells (as determined e.g. by immunohistochemistry in tumor biopsies).

The herein mentioned "patient" is a mammal, preferably a human, who suffers from cancer (particularly a solid tumor cancer) and will be or (already) is treated with a CD3 antibody. In particular embodiments the cancer is a solid tumor cancer.

By a "solid tumor cancer" is meant a malignancy that forms a discrete tumor mass (including also tumor metastasis) located at specific location in the patient's body, such as sarcomas or carcinomas (as opposed to e.g. blood cancers such as leukemia, which generally do not form solid tumors). Non-limiting examples of solid tumor cancers include bladder cancer, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, skin cancer, squamous cell carcinoma, bone cancer, liver cancer and kidney cancer. Other solid tumor cancers that are contemplated in the context of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous system (central and peripheral), lymphatic system, pelvic, skin, soft tissue, muscles, spleen, thoracic region, and urogenital system. Also included are pre-cancerous conditions or lesions and cancer metastases.

In certain embodiments the cancer is a cancer selected from the group consisting of colorectal cancer, lung cancer, pancreatic cancer, breast cancer, and gastric cancer. In a particular embodiment, the cancer is a colorectal cancer.

In some embodiments wherein the CD3 antibody is a bispecific antibody that specifically binds to CD3 and to a target cell antigen, the cancer expresses said target cell antigen. Exemplary target cell antigens are described herein. In one embodiment, the cancer expresses CEA. In one embodiment, the cancer expresses CEA in at least 20%, preferably at least 50% or at least 80% of tumor cells as determined by immunohistochemistry (IHC) using an antibody specific for CEA.

Glucocorticoids (GCs, sometimes also referred to as glucocorticosteroids) are widely used immunosuppressive agents for the treatment of inflammatory disorders and autoimmune diseases. GCs are a class of steroid hormones that bind to the glucocorticoid receptor, which is present in almost every vertebrate animal cell, including humans. These compounds are potent antiinflammatory agents, regardless of the inflammation's cause.

As used herein, the term "glucocorticoid" means compounds that bind, preferably specifically, to the glucocorticoid receptor. Said term includes, but is not limited to, compound(s) selected from the group consisting of cortisone, cortisol (hydrocortisone), cloprednol, prednisone, prednisolone, methylprednisolone, deflazacort, fluocortolone, triamcinolone, (including triamcinolonacetonide), dexamethasone, betamethasone, cortivazol, paramethasone, prednylidene, and/or fluticasone (including fluticasonepropionate), including pharmaceutically acceptable derivatives thereof.

The term "pharmaceutically acceptable derivatives" includes salts, esters, enol ethers, enol esters, acetals, ketals, orthoesters, hemiacetals, hemiketals, acids, bases, solvates, hydrates or prodrugs thereof. Such derivatives may be readily prepared by those of skill in the art using known methods for such derivatisation. In the context of the embodiments of the present invention, the mentioned compounds may be used alone or in combination. The present invention is however not limited to the above mentioned specific GCs. It is envisaged that all substances which already are or will be classified as a "glucocorticoid", may be employed in the context of the present invention as well. Such future glucocorticoids include compounds which specifically bind to and activate the glucocorticoid receptor. The term "specifically binds to the GC receptor" means in accordance with the present invention that the GC (or a compound which is assumed to act like a GC) associates with (e.g., interacts with) the GC receptor (also known as NR3C1) to a statistically significant degree as compared to association with proteins/receptors generally (i.e., non-specific binding). When the GC receptor binds to glucocorticoids, its primary mechanism of action is the regulation of gene transcription. In the absence of GC, the glucocorticoid receptor resides in the cytosol complexed with a variety of proteins including heat shock protein 90 (hsp90), heat shock protein 70 (hsp70) and the protein FKBP52 (FK506-binding protein 52). The binding of the GC to the glucocorticoid receptor results in release of the heat shock proteins. It is thus envisaged that a future GC, or a pharmaceutically acceptable derivative or salt of a GC, is preferably able to bind to the GC receptor and to release the above mentioned heat shock proteins. The activated glucocorticoid receptor complex up-regulates the expression of antiinflammatory proteins in the nucleus or represses the expression of pro-inflammatory proteins in the cytosol (by preventing the translocation of other transcription factors from the cytosol into the nucleus).

In particular embodiments according to the present invention, the GC is selected from the most clinically used and relevant GCs like prednisone, prednisolone, methylprednisolone, betamethasone, dexamethasone, fluticasonepropionate, triamcinolonacetonide, cortisone, hydrocortisone or combinations thereof. GCs for systemic administration are particularly suitable. GCs for intravenuous and/or oral administration are particularly suitable.

In one embodiment, the GC is selected from the group consisting of methylprednisolone, prednisone, and prednisolone. In a particular embodiment, the GC is methylprednisolone or prednisone. In one embodiment, the GC is methylprednisolone. In one embodiment, the GC is prednisone. In one embodiment, the GC is prednisolone. In one embodiment, the GC is not dexamethasone.

Prednisone and methylprednisolone are particularly suitable in the context of the present invention due to their intermediate half-life (among glucocorticoids) that allows for a daily dose without accumulation, and also due to their availability for intravenous as well as oral administration. A person skilled in the field, however, can select one of the other known glucocorticoids, some of which are disclosed herein, and select an appropriate effective dose to ameliorate, treat or prevent tumor inflammation-related adverse effects that may result from the treatment of a patient in need thereof, such as a cancer patient, with a (bispecific) CD3 antibody, such as CEAxCD3 bispecific antibody.

The GC is preferably administered in an amount which is sufficient to ameliorate, treat or prevent said tumor inflammation-related adverse effects caused by the CD3 antibody. The tumor inflammation-related adverse effects are "caused by" the administration of a CD3 antibody to a patient. The term "caused by" means that the CD3 antibody is causative for the adverse effects. The skilled person can easily evaluate whether the administration a CD3 binding domain is causative for an adverse effect or not, e.g. by closely monitoring the patient during the course of the administration and to detect, thereby, that the administration of the CD3 antibody was causative for the adverse effects. Likewise, it is envisaged to discontinue the administration of the CD3 antibody and to evaluate whether the adverse effects are thereby ameliorated or even fade away, which also indicates that they were caused by said CD3 antibody.

The dose of the GC that is to be used in accordance with the embodiments of the present invention is not limited, i.e. it will depend on the circumstances of the individual patient. The GC can be administered intravenously or orally. Preferred dosages of the GC include, however, between about 5 mg to about 60 mg, e.g. about 40 mg (prednisone equivalent). Said dosage can be administered all at once or subdivided into smaller dosages. Particularly preferred is a dosage of about 40 mg (prednisone equivalent).

In particular embodiments, about 40 mg of predisone or about 40 mg of methylprednisone is administered intravenously or orally.

In the context of the present invention, the GC may be given prior to, concurrently with, or after the administration(s) of the CD3 antibody. In preferred embodiments, the GC is given after the administration of the CD3 antibody.

The GC may be given after each administration (if several) of the CD3 antibody. In particular embodiments, the GC is given after the first administration of the CD3 antibody. In some embodiments, the GC is given only after the first (i.e. not after a subsequent) administration of the CD3 antibody. In some embodiments, the GC is given only after the first and the after the second (i.e. not after a subsequent) administration of the CD3 antibody.

In some embodiments, the GC is administered in the range of between about 1 hour and about 7 days after the (first) administration of the CD3 antibody, particularly between about 1 and about 72 hours, after the (first) administration of the CD3 antibody. In some embodiments, more than one dose of GC (particularly two doses, four doses, or eight doses) is administered in the above time range. Each of the GC doses is preferably between 5 and 60 mg (prednisone equivalent), and preferably about 40 mg per dose. In particular embodiments, each dose is about 40 mg of predisone or about 40 mg of methylprednisone.

The time range between about 1 hour and about 7 days means that the time after the (first) administration of the CD3 antibody may be about 1, 2, 3, 4, 5, 6, 7, 8, 9, 10, 1 1, 12, 13, 14, 15, 16, 17, 18, 19, 20, 21, 22, 23, 24 hours, 1 day, 2 days, 3 days, 4 days, 5 days, 6 days or 7 days, e.g. about 3 hours, about 24 hours, about 2 days, about 3 days or about 7 days.

The time range between about 1 and about 72 hours means that the time after the (first) administration of the CD3 antibody may be about 1, 2, 3, 4, 5, 6, 7, 8, 9, 10, 1 1, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71 or 72 hours, e.g. about 3 hours, about 24 hours, about 48 hours or about 72 hours.

In particular embodiments, the GC is administered at about 3 hours, at about 1 day (24 hours), at about 2 days (48 hours), and at about 3 days (72 hours) after the (first) administration of the CD3 antibody. In other particular embodiments, the GC is administered at about 3 hours, at about 1 day (24 hours), at about 2 days (48 hours), at about 3 days (72 hours), at about 4 days (96 hours), at about 5 days (120 hours), at about 6 days (144 hours) and at about 7 days (168 hours) after the (first) administration of the CD3 antibody.

In a specific embodiment, 40 mg of methylprednisolone or 40 mg of prednisone is administered at about 3 hours, at about 1 day (24 hours), at about 2 days (48 hours), and at about 3 days (72 hours) after the (first) administration of the CD3 antibody. In another particular embodiment, 40 mg of methylprednisolone or 40 mg of prednisone is administered at about 3 hours, at about 1 day (24 hours), at about 2 days (48 hours), at about 3 days (72 hours), at about 4 days (96 hours), at about 5 days (120 hours), at about 6 days (144 hours) and at about 7 days (168 hours) after the (first) administration of the CD3 antibody.

The duration of the GC administration (e.g. up to 3 days or up to 7 days) may depend on the treatment regimen applied for the CD3 antibody, e.g. the dose of the CD3 antibody, or its combination with further therapeutic agents that may enhance tumor inflammation.

In some embodiments, the CD3 antibody is used as monotherapy (i.e. no further anti-cancer therapeutic agent is used together with the CD3 antibody). In one such embodiment, the GC is administered at about 3 hours, at about 1 day (24 hours), at about 2 days, and at about 3 days after the (first) administration of the CD3 antibody.

In other embodiments, the CD3 antibody is used in a combination treatment with a further therapeutic agent, wherein said further therapeutic agent may cause tumor inflammation-related adverse effects and/or enhance tumor inflammation-related adverse effects caused by the CD3 antibody. In one such embodiment, the GC is administered at about 3 hours, at about 1 day (24 hours), at about 2 days, at about 3 days, at about 4 days, at about 5 days, at about 6 days and at about 7 days after the (first) administration of the CD3 antibody.

In one embodiment, the CD3 antibody is used in combination with a PD-1 axis binding antagonist, particularly a human PD-1 axis binding antagonist. The term "PD-1 axis binding antagonist" refers to a molecule that inhibits the interaction of a PD-1 axis binding partner with either one or more of its binding partner, so as to remove T-cell dysfunction resulting from signaling on the PD-1 signaling axis - with a result being to restore or enhance T-cell function (e.g., proliferation, cytokine production, target cell killing). As used herein, a PD-1 axis binding antagonist includes a PD-1 binding antagonist, a PD-L1 binding antagonist and a PD-L2 binding antagonist. A "human" PD-1 axis binding antagonist refers to a PD-1 axis binding antagonist which has the above-described effects on the human PD-1 signaling axis.

In some embodiments the PD-1 axis binding antagonist is selected from the group consisting of a PD-1 binding antagonist, a PD-L1 binding antagonist and a PD-L2 binding antagonist. The term "PD-1 binding antagonist" refers to a molecule that decreases, blocks, inhibits, abrogates or interferes with signal transduction resulting from the interaction of PD-1 with one or more of its binding partners, such as PD-L1, PD-L2. In some embodiments, the PD-1 binding antagonist is a molecule that inhibits the binding of PD-1 to one or more of its binding partners. In a specific aspect, the PD-1 binding antagonist inhibits the binding of PD-1 to PD-L1 and/or PD-L2. For example, PD-1 binding antagonists include anti-PD-1 antibodies, antigen binding fragments thereof, immunoadhesins, fusion proteins, oligopeptides and other molecules that decrease, block, inhibit, abrogate or interfere with signal transduction resulting from the interaction of PD-1 with PD-L1 and/or PD-L2. In one embodiment, a PD-1 binding antagonist reduces the negative co-stimulatory signal mediated by or through cell surface proteins expressed on T lymphocytes mediated signaling through PD-1 so as to render a dysfunctional T-cell less dysfunctional (e.g., enhancing effector responses to antigen recognition). In some embodiments, the PD-1 binding antagonist is an anti-PD-1 antibody. In a specific aspect, a PD-1 binding antagonist is MDX-1106 (nivolumab). In another specific aspect, a PD-1 binding antagonist is MK-3475 (pembrolizumab). In another specific aspect, a PD-1 binding antagonist is CT-011 (pidilizumab). In another specific aspect, a PD-1 binding antagonist is MEDI-0680 (AMP-514) described herein. In another specific aspect, a PD-1 binding antagonist is PDR001. In another specific aspect, a PD-1 binding antagonist is REGN2810. In another specific aspect, a PD-1 binding antagonist is BGB-108. The term "PD-L1 binding antagonist" refers to a molecule that decreases, blocks, inhibits, abrogates or interferes with signal transduction resulting from the interaction of PD-L1 with either one or more of its binding partners, such as PD-1, B7-1. In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some embodiments, the PD-L1 binding antagonists include anti-PD-L1 antibodies, antigen binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1, B7-1. In one embodiment, a PD-L1 binding antagonist reduces the negative co-stimulatory signal mediated by or through cell surface proteins expressed on T lymphocytes mediated signaling through PD-L1 so as to render a dysfunctional T-cell less dysfunctional (e.g., enhancing effector responses to antigen recognition). In some embodiments, a PD-L1 binding antagonist is an anti-PD-L1 antibody. In a specific aspect, an anti-PD-L1 antibody is YW243.55.S70. In another specific aspect, an anti-PD-L1 antibody is MDX-1105. In still another specific aspect, an anti-PD-L1 antibody is MPDL3280A (atezolizumab). In still another specific aspect, an anti-PD-L1 antibody is MDX-1105. In still another specific aspect, an anti-PD-L1 antibody is MEDI4736 (durvalumab). In still another specific aspect, an anti-PD-L1 antibody is MSB0010718C (avelumab). The term "PD-L2 binding antagonist" refers to a molecule that decreases, blocks, inhibits, abrogates or interferes with signal transduction resulting from the interaction of PD-L2 with either one or more of its binding partners, such as PD-1. In some embodiments, a PD-L2 binding antagonist is a molecule that inhibits the binding of PD-L2 to one or more of its binding partners. In a specific aspect, the PD-L2 binding antagonist inhibits binding of PD-L2 to PD-1. In some embodiments, the PD-L2 antagonists include anti-PD-L2 antibodies, antigen binding fragments thereof, immunoadhesins, fusion proteins, oligopeptides and other molecules that decrease, block, inhibit, abrogate or interfere with signal transduction resulting from the interaction of PD-L2 with either one or more of its binding partners, such as PD-1. In one embodiment, a PD-L2 binding antagonist reduces the negative co-stimulatory signal mediated by or through cell surface proteins expressed on T lymphocytes mediated signaling through PD-L2 so as render a dysfunctional T-cell less dysfunctional (e.g., enhancing effector responses to antigen recognition). In some embodiments, a PD-L2 binding antagonist is an immunoadhesin.

In some embodiments, the PD-1 axis binding antagonist is an antibody. In some embodiments, the antibody is a humanized antibody, a chimeric antibody or a human antibody. In some embodiments, the antibody is an antigen binding fragment. In some embodiments, the antigen-binding fragment is selected from the group consisting of Fab, Fab', F(ab')₂, and Fv.

In some embodiments, the PD-1 axis binding antagonist is a PD-1 binding antagonist. In some embodiments, the PD-1 binding antagonist inhibits the binding of PD-1 to its ligand binding partners. In some embodiments, the PD-1 binding antagonist inhibits the binding of PD-1 to PD-L1. In some embodiments, the PD-1 binding antagonist inhibits the binding of PD-1 to PD-L2. In some embodiments, the PD-1 binding antagonist inhibits the binding of PD-1 to both PD-L1 and PD-L2. In some embodiments, the PD-1 binding antagonist is an antibody. In some embodiments, the PD-1 binding antagonist is selected from the group consisting of MDX 1106 (nivolumab), MK-3475 (pembrolizumab), CT-011 (pidilizumab), MEDI-0680 (AMP-514), PDR001, REGN2810, and BGB-108.

In some embodiments, the PD-1 axis binding antagonist is a PD-L1 binding antagonist. In some embodiments, the PD-L1 binding antagonist inhibits the binding of PD-L1 to PD-1. In some embodiments, the PD-L1 binding antagonist inhibits the binding of PD-L1 to B7-1. In some embodiments, the PD-L1 binding antagonist inhibits the binding of PD-L1 to both PD-1 and B7-1. In some embodiments, the PD-L1 binding antagonist is an anti-PD-L1 antibody. In some embodiments, the PD-L1 binding antagonist is selected from the group consisting of: MPDL3280A (atezolizumab), YW243.55.S70, MDX-1105, MEDI4736 (durvalumab), and MSB0010718C (avelumab).

In a preferred embodiment, the PD-1 axis binding antagonist is atezolizumab. In some embodiments, atezolizumab is administered at a dose of about 800 mg to about 1500 mg every three weeks (e.g., about 1000 mg to about 1300 mg every three weeks, e.g., about 1100 mg to about 1200 mg every three weeks). In a preferred embodiment, atezolizumab is administered at a dose of about 1200 mg every three weeks.

The CD3 antibody used in the present invention specifically binds to CD3, particularly CD3ε, most particularly human CD3 / CD3ε (see SEQ ID NO: 34).

In one embodiment, the CD3 antibody is or can compete for binding with antibody H2C (PCT publication no. WO2008/119567), antibody V9 (Rodrigues et al., Int J Cancer Suppl 7, 45-50 (1992) and US patent no. 6,054,297), antibody FN18 (Nooij et al., Eur J Immunol 19, 981-984 (1986)), antibody SP34 (Pessano et al., EMBO J 4, 337-340 (1985)), antibody OKT3 (Kung et al., Science 206, 347-349 (1979)), antibody WT31 (Spits et al., J Immunol 135, 1922 (1985)), antibody UCHT1 (Burns et al., J Immunol 129, 1451-1457 (1982)), antibody 7D6 (Coulie et al., Eur J Immunol 21, 1703-1709 (1991)), antibody Leu-4, or antibody Cris-7 (Reinherz et al. (eds.), Leukocyte Typing II., Springer Verlag, New York, (1986)). In some embodiments, the CD3 antibody may also be an antibody that specifically binds to CD3 as described in WO 2005/040220, WO 2005/118635, WO 2007/042261, WO 2008/119567, WO 2008/119565, WO 2012/162067, WO 2013/158856, WO 2013/188693, WO 2013/186613, WO 2014/110601, WO 2014/145806, WO 2014/191113, WO 2014/047231, WO 2015/095392, WO 2015/181098, WO 2015/001085, WO 2015/104346, WO 2015/172800, WO 2016/071004, WO 2016/116626, WO 2016/166629, WO 2016/020444, WO 2016/014974, WO 2016/204966, WO 2017/009442, WO 2017/53469, WO 2017/010874, or WO 2017/53856.

In one embodiment, the CD3 antibody comprises a heavy chain variable region comprising the heavy chain CDR (HCDR) 1 of SEQ ID NO: 1, the HCDR2 of SEQ ID NO: 2, and the HCDR3 of SEQ ID NO: 3; and a light chain variable region comprising the light chain CDR (LCDR) 1 of SEQ ID NO: 4, the LCDR2 of SEQ ID NO: 5 and the LCDR3 of SEQ ID NO: 6.

In one embodiment, the CD3 antibody comprises a heavy chain variable region sequence that is at least about 95%, 96%, 97%, 98%, 99% or 100% identical to the amino acid sequence of SEQ ID NO: 7 and a light chain variable region sequence that is at least about 95%, 96%, 97%, 98%, 99% or 100% identical to the amino acid sequence of SEQ ID NO: 8.

In one embodiment, the CD3 antibody comprises the heavy chain variable region sequence of SEQ ID NO: 7 and the light chain variable region sequence of SEQ ID NO: 8.

In one embodiment, the CD3 antibody is an IgG, particularly an IgG₁, antibody. In one embodiment, the antibody is a full-length antibody. In another embodiment, the antibody is an antibody fragment selected from the group of an Fv molecule, a scFv molecule, a Fab molecule, and a F(ab')₂ molecule.

In some embodiments, the CD3 antibody provides monovalent binding to CD3.

In particular embodiments, the CD3 antibody is a multispecific antibody, e.g. a bispecific antibody. In one embodiment, the CD3 antibody is a bispecific antibody that specifically binds to CD3 and to a target cell (e.g. a tumor cell) antigen. Suitable target cell antigens include in particular antigens expressed on solid tumors and may include, for example, carcinoembryonic antigen (CEA), epithelial cell adhesion molecule (EpCAM), Her2, Her3, epidermal growth factor receptor (EGFR), EGFRvIII, ganglioside GD2, CD44v6, six-transmembrane epithelial antigen of prostate 1 (STEAP-1), mesothelin (MSLN), melanoma-associated chondroitin sulfate proteoglycan (MCSP), fibroblast activation protein (FAP), 5T4 oncofetal antigen, Trop2, cadherin 19 (CDH19), CDH3, P-cadherin, Fc receptor-like 5 (FcRH5), glypican 3 (GPC3), claudin (CLDN), B7-H3, prostate-specific membrane antigen (PSMA), insulin-like growth factor 1 receptor (IGF-1R), prostate stem cell antigen (PSCA), c-Met, glycoprotein A33 (gpA33), death receptor 5 (DR5), ephrin A2 (EphA2), and delta-like 3 (DLL3).

In some embodiments, the CD3 antibody is a bispecific antibody directed against CD3 and EpCAM. In one embodiment, the bispecific antibody is catumaxomab (REVOMAB®). In one embodiment, the bispecific antibody is solitomab (AMG 110, MT110). In some embodiments, the CD3 antibody is a bispecific antibody directed against CD3 and Her2. In one embodiment, the bispecific antibody is ertumaxomab. In one embodiment, the bispecific antibody is GBR 1302. In some embodiments, the CD3 antibody is a bispecific antibody directed against CD3 and PSMA. In one embodiment, the bispecific antibody is BAY2010112 (AMG212, MT112). In one embodiment, the bispecific antibody is MOR209/ES414. In some embodiments, the CD3 antibody is a bispecific antibody directed against CD3 and CEA. In one embodiment, the bispecific antibody is MEDI565 (AMG211, MT111). In some embodiments, the CD3 antibody is a bispecific antibody directed against CD3 and gpA33. In one embodiment, the bispecific antibody is MGD007. In some embodiments, the CD3 antibody is a bispecific antibody directed against CD3 and GPC3. In one embodiment, the bispecific antibody is ERY974. In some embodiments, the CD3 antibody is a bispecific antibody directed against CD3 and P-cadherin. In one embodiment, the bispecific antibody is PF-06671008. In some embodiments, the CD3 antibody is a bispecific antibody directed against CD3 and B7-H4. In one embodiment, the bispecific antibody is MGD009.

In particular embodiments, the CD3 antibody is a bispecific antibody that specifically binds to CD3 and to CEA. Particular bispecific antibodies directed to CD3 and CEA are described e.g. in PCT publication nos. WO 2014/131712 and WO 2017/055389 (each incorporated herein by reference in its entirety).

In one embodiment the CD3 antibody is a bispecific antibody comprising
(i) a first antigen binding moiety that specifically binds to CD3 and comprises a heavy chain variable region comprising the heavy chain CDR (HCDR) 1 of SEQ ID NO: 1, the HCDR2 of SEQ ID NO: 2, and the HCDR3 of SEQ ID NO: 3; and a light chain variable region comprising the light chain CDR (LCDR) 1 of SEQ ID NO: 4, the LCDR2 of SEQ ID NO: 5 and the LCDR3 of SEQ ID NO: 6; and
(ii) a second antigen binding moiety that specifically binds to CEA and comprises (i) a heavy chain variable region comprising the heavy chain CDR (HCDR) 1 of SEQ ID NO: 9, the HCDR2 of SEQ ID NO: 10, and the HCDR3 of SEQ ID NO: 11; and a light chain variable region comprising the light chain CDR (LCDR) 1 of SEQ ID NO: 12, the LCDR2 of SEQ ID NO: 13 and the LCDR3 of SEQ ID NO: 14; or (ii) a heavy chain variable region comprising the heavy chain CDR (HCDR) 1 of SEQ ID NO: 17, the HCDR2 of SEQ ID NO: 18, and the HCDR3 of SEQ ID NO: 19; and a light chain variable region comprising the light chain CDR (LCDR) 1 of SEQ ID NO: 20, the LCDR2 of SEQ ID NO: 21 and the LCDR3 of SEQ ID NO: 22.

In one embodiment, the first antigen binding moiety comprises a heavy chain variable region sequence that is at least about 95%, 96%, 97%, 98%, 99% or 100% identical to the amino acid sequence of SEQ ID NO: 7 and a light chain variable region sequence that is at least about 95%, 96%, 97%, 98%, 99% or 100% identical to the amino acid sequence of SEQ ID NO: 8.

In one embodiment, the first antigen binding moiety comprises the heavy chain variable region sequence of SEQ ID NO: 7 and the light chain variable region sequence of SEQ ID NO: 8.

In one embodiment, the second antigen binding moiety comprises (i) a heavy chain variable region sequence that is at least about 95%, 96%, 97%, 98%, 99% or 100% identical to the amino acid sequence of SEQ ID NO: 15 and a light chain variable region sequence that is at least about 95%, 96%, 97%, 98%, 99% or 100% identical to the amino acid sequence of SEQ ID NO: 16; or (ii) a heavy chain variable region sequence that is at least about 95%, 96%, 97%, 98%, 99% or 100% identical to the amino acid sequence of SEQ ID NO: 23 and a light chain variable region sequence that is at least about 95%, 96%, 97%, 98%, 99% or 100% identical to the amino acid sequence of SEQ ID NO: 24.

In one embodiment, the second antigen binding moiety comprises (i) the heavy chain variable region sequence of SEQ ID NO: 15 and the light chain variable region sequence of SEQ ID NO: 16; or (ii) the heavy chain variable region sequence of SEQ ID NO: 23 and the light chain variable region sequence of SEQ ID NO: 24.

In some embodiments, the first and/or the second antigen binding moiety is a Fab molecule. In some embodiments, the first antigen binding moiety is a crossover Fab molecule wherein either the variable or the constant regions of the Fab light chain and the Fab heavy chain are exchanged. In such embodiments, the second antigen binding moiety preferably is a conventional Fab molecule.

In some embodiments wherein the first and the second antigen binding moiety of the bispecific antigen binding molecule are both Fab molecules, and in one of the antigen binding moieties (particularly the first antigen binding moiety) the variable domains VL and VH of the Fab light chain and the Fab heavy chain are replaced by each other,
i) in the constant domain CL of the first antigen binding moiety the amino acid at position 124 is substituted by a positively charged amino acid (numbering according to Kabat), and wherein in the constant domain CH1 of the first antigen binding moiety the amino acid at position 147 or the amino acid at position 213 is substituted by a negatively charged amino acid (numbering according to Kabat EU index); or
ii) in the constant domain CL of the second antigen binding moiety the amino acid at position 124 is substituted by a positively charged amino acid (numbering according to Kabat), and wherein in the constant domain CH1 of the second antigen binding moiety the amino acid at position 147 or the amino acid at position 213 is substituted by a negatively charged amino acid (numbering according to Kabat EU index).

The bispecific antibody does not comprise both modifications mentioned under i) and ii). The constant domains CL and CH1 of the antigen binding moiety having the VH/VL exchange are not replaced by each other (i.e. remain unexchanged).

In a more specific embodiment,
i) in the constant domain CL of the first antigen binding moiety the amino acid at position 124 is substituted independently by lysine (K), arginine (R) or histidine (H) (numbering according to Kabat), and in the constant domain CH1 of the first antigen binding moiety the amino acid at position 147 or the amino acid at position 213 is substituted independently by glutamic acid (E), or aspartic acid (D) (numbering according to Kabat EU index); or
ii) in the constant domain CL of the second antigen binding moiety the amino acid at position 124 is substituted independently by lysine (K), arginine (R) or histidine (H) (numbering according to Kabat), and in the constant domain CH1 of the second antigen binding moiety the amino acid at position 147 or the amino acid at position 213 is substituted independently by glutamic acid (E), or aspartic acid (D) (numbering according to Kabat EU index).

In one such embodiment, in the constant domain CL of the second antigen binding moiety the amino acid at position 124 is substituted independently by lysine (K), arginine (R) or histidine (H) (numbering according to Kabat), and in the constant domain CH1 of the second antigen binding moiety the amino acid at position 147 or the amino acid at position 213 is substituted independently by glutamic acid (E), or aspartic acid (D) (numbering according to Kabat EU index).

In a further embodiment, in the constant domain CL of the second antigen binding moiety the amino acid at position 124 is substituted independently by lysine (K), arginine (R) or histidine (H) (numbering according to Kabat), and in the constant domain CH1 of the second antigen binding moiety the amino acid at position 147 is substituted independently by glutamic acid (E), or aspartic acid (D) (numbering according to Kabat EU index).

In a particular embodiment, in the constant domain CL of the second antigen binding moiety the amino acid at position 124 is substituted independently by lysine (K), arginine (R) or histidine (H) (numbering according to Kabat) and the amino acid at position 123 is substituted independently by lysine (K), arginine (R) or histidine (H) (numbering according to Kabat), and in the constant domain CH1 of the second antigen binding moiety the amino acid at position 147 is substituted independently by glutamic acid (E), or aspartic acid (D) (numbering according to Kabat EU index) and the amino acid at position 213 is substituted independently by glutamic acid (E), or aspartic acid (D) (numbering according to Kabat EU index).

In a more particular embodiment, in the constant domain CL of the second antigen binding moiety the amino acid at position 124 is substituted by lysine (K) (numbering according to Kabat) and the amino acid at position 123 is substituted by lysine (K) (numbering according to Kabat), and in the constant domain CH1 of the second antigen binding moiety the amino acid at position 147 is substituted by glutamic acid (E) (numbering according to Kabat EU index) and the amino acid at position 213 is substituted by glutamic acid (E) (numbering according to Kabat EU index).

In an even more particular embodiment, in the constant domain CL of the second antigen binding moiety the amino acid at position 124 is substituted by lysine (K) (numbering according to Kabat) and the amino acid at position 123 is substituted by arginine (R) (numbering according to Kabat), and in the constant domain CH1 of the second antigen binding moiety the amino acid at position 147 is substituted by glutamic acid (E) (numbering according to Kabat EU index) and the amino acid at position 213 is substituted by glutamic acid (E) (numbering according to Kabat EU index).

In particular embodiments, if amino acid substitutions according to the above embodiments are made in the constant domain CL and the constant domain CH1 of the second antigen binding moiety, the constant domain CL of the second antigen binding moiety is of kappa isotype.

In some embodiments, the first and the second antigen binding moiety are fused to each other, optionally via a peptide linker.

In some embodiments, the first and the second antigen binding moiety are each a Fab molecule and either (i) the second antigen binding moiety is fused at the C-terminus of the Fab heavy chain to the N-terminus of the Fab heavy chain of the first antigen binding moiety, or (ii) the first antigen binding moiety is fused at the C-terminus of the Fab heavy chain to the N-terminus of the Fab heavy chain of the second antigen binding moiety.

In particular embodiments, the bispecific antibody comprises a single antigen binding moiety that specifically binds to CD3, and two antigen binding moieties that specifically bind to CEA. Thus, in some embodiments, the bispecific antigen binding molecule comprises a third antigen binding moiety that specifically binds to CEA. In some embodiments, the third antigen moiety is identical to the first antigen binding moiety (e.g. is also a Fab molecule and comprises the same amino acid sequences).

In particular embodiments, the bispecific antibody further comprises an Fc domain composed of a first and a second subunit. In one embodiment, the Fc domain is an IgG Fc domain. In a particular embodiment, the Fc domain is an IgG₁ Fc domain. In another embodiment the Fc domain is an IgG₄ Fc domain. In a more specific embodiment, the Fc domain is an IgG₄ Fc domain comprising an amino acid substitution at position S228 (Kabat EU index numbering), particularly the amino acid substitution S228P. This amino acid substitution reduces *in vivo* Fab arm exchange of IgG₄ antibodies (see Stubenrauch et al., Drug Metabolism and Disposition 38, 84-91 (2010)). In a further particular embodiment, the Fc domain is a human Fc domain. In an even more particular embodiment, the Fc domain is a human IgG₁ Fc domain. An exemplary sequence of a human IgG₁ Fc region is given in SEQ ID NO: 33.

In some embodiments wherein the first, the second and, where present, the third antigen binding moiety are each a Fab molecule, (a) either (i) the second antigen binding moiety is fused at the C-terminus of the Fab heavy chain to the N-terminus of the Fab heavy chain of the first antigen binding moiety and the first antigen binding moiety is fused at the C-terminus of the Fab heavy chain to the N-terminus of the first subunit of the Fc domain, or (ii) the first antigen binding moiety is fused at the C-terminus of the Fab heavy chain to the N-terminus of the Fab heavy chain of the second antigen binding moiety and the second antigen binding moiety is fused at the C-terminus of the Fab heavy chain to the N-terminus of the first subunit of the Fc domain; and (b) the third antigen binding moiety, where present, is fused at the C-terminus of the Fab heavy chain to the N-terminus of the second subunit of the Fc domain.

In particular embodiments, the Fc domain comprises a modification promoting the association of the first and the second subunit of the Fc domain. The site of most extensive protein-protein interaction between the two subunits of a human IgG Fc domain is in the CH3 domain. Thus, in one embodiment said modification is in the CH3 domain of the Fc domain.

In a specific embodiment said modification promoting the association of the first and the second subunit of the Fc domain is a so-called "knob-into-hole" modification, comprising a "knob" modification in one of the two subunits of the Fc domain and a "hole" modification in the other one of the two subunits of the Fc domain. The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in some embodiments, an amino acid residue in the CH3 domain of the first subunit of the Fc domain is replaced with an amino acid residue having a larger side chain volume, thereby generating a protuberance within the CH3 domain of the first subunit which is positionable in a cavity within the CH3 domain of the second subunit, and an amino acid residue in the CH3 domain of the second subunit of the Fc domain is replaced with an amino acid residue having a smaller side chain volume, thereby generating a cavity within the CH3 domain of the second subunit within which the protuberance within the CH3 domain of the first subunit is positionable. Preferably said amino acid residue having a larger side chain volume is selected from the group consisting of arginine (R), phenylalanine (F), tyrosine (Y), and tryptophan (W). Preferably said amino acid residue having a smaller side chain volume is selected from the group consisting of alanine (A), serine (S), threonine (T), and valine (V). The protuberance and cavity can be made by altering the nucleic acid encoding the polypeptides, e.g. by site-specific mutagenesis, or by peptide synthesis.

In a specific such embodiment, in the first subunit of the Fc domain the threonine residue at position 366 is replaced with a tryptophan residue (T366W), and in the second subunit of the Fc domain the tyrosine residue at position 407 is replaced with a valine residue (Y407V) and optionally the threonine residue at position 366 is replaced with a serine residue (T366S) and the leucine residue at position 368 is replaced with an alanine residue (L368A) (numbering according to Kabat EU index). In a further embodiment, in the first subunit of the Fc domain additionally the serine residue at position 354 is replaced with a cysteine residue (S354C) or the glutamic acid residue at position 356 is replaced with a cysteine residue (E356C) (particularly the serine residue at position 354 is replaced with a cysteine residue), and in the second subunit of the Fc domain additionally the tyrosine residue at position 349 is replaced by a cysteine residue (Y349C) (numbering according to Kabat EU index). In a particular embodiment, the first subunit of the Fc domain comprises the amino acid substitutions S354C and T366W, and the second subunit of the Fc domain comprises the amino acid substitutions Y349C, T366S, L368A and Y407V (numbering according to Kabat EU index).

In some embodiments, the Fc domain comprises one or more amino acid substitution that reduces binding to an Fc receptor and/or effector function.

In a particular embodiment the Fc receptor is an Fcγ receptor. In one embodiment the Fc receptor is a human Fc receptor. In one embodiment the Fc receptor is an activating Fc receptor. In a specific embodiment the Fc receptor is an activating human Fcγ receptor, more specifically human FcγRIIIa, FcγRI or FcγRIIa, most specifically human FcγRIIIa. In one embodiment the effector function is one or more selected from the group of complement dependent cytotoxicity (CDC), antibody-dependent cell-mediated cytotoxicity (ADCC), antibody-dependent cellular phagocytosis (ADCP), and cytokine secretion. In a particular embodiment, the effector function is ADCC.

Typically, the same one or more amino acid substitution is present in each of the two subunits of the Fc domain. In one embodiment, the one or more amino acid substitution reduces the binding affinity of the Fc domain to an Fc receptor. In one embodiment, the one or more amino acid substitution reduces the binding affinity of the Fc domain to an Fc receptor by at least 2-fold, at least 5-fold, or at least 10-fold.

In one embodiment, the Fc domain comprises an amino acid substitution at a position selected from the group of E233, L234, L235, N297, P331 and P329 (numberings according to Kabat EU index). In a more specific embodiment, the Fc domain comprises an amino acid substitution at a position selected from the group of L234, L235 and P329 (numberings according to Kabat EU index). In some embodiments, the Fc domain comprises the amino acid substitutions L234A and L235A (numberings according to Kabat EU index). In one such embodiment, the Fc domain is an IgG₁ Fc domain, particularly a human IgG₁ Fc domain. In one embodiment, the Fc domain comprises an amino acid substitution at position P329. In a more specific embodiment, the amino acid substitution is P329A or P329G, particularly P329G (numberings according to Kabat EU index). In one embodiment, the Fc domain comprises an amino acid substitution at position P329 and a further amino acid substitution at a position selected from E233, L234, L235, N297 and P331 (numberings according to Kabat EU index). In a more specific embodiment, the further amino acid substitution is E233P, L234A, L235A, L235E, N297A, N297D or P331S. In particular embodiments, the Fc domain comprises amino acid substitutions at positions P329, L234 and L235 (numberings according to Kabat EU index). In more particular embodiments, the Fc domain comprises the amino acid mutations L234A, L235A and P329G ("P329G LALA", "PGLALA" or "LALAPG"). Specifically, in particular embodiments, each subunit of the Fc domain comprises the amino acid substitutions L234A, L235A and P329G (Kabat EU index numbering), i.e. in each of the first and the second subunit of the Fc domain the leucine residue at position 234 is replaced with an alanine residue (L234A), the leucine residue at position 235 is replaced with an alanine residue (L235A) and the proline residue at position 329 is replaced by a glycine residue (P329G) (numbering according to Kabat EU index). In one such embodiment, the Fc domain is an IgG₁ Fc domain, particularly a human IgG₁ Fc domain.

In one embodiment the CD3 antibody is a bispecific antibody comprising
(i) a first antigen binding moiety that specifically binds to CD3, comprising a heavy chain variable region comprising the heavy chain CDR (HCDR) 1 of SEQ ID NO: 1, the HCDR2 of SEQ ID NO: 2, and the HCDR3 of SEQ ID NO: 3; and a light chain variable region comprising the light chain CDR (LCDR) 1 of SEQ ID NO: 4, the LCDR2 of SEQ ID NO: 5 and the LCDR3 of SEQ ID NO: 6, wherein the first antigen binding moiety is a crossover Fab molecule wherein either the variable or the constant regions, particularly the constant regions, of the Fab light chain and the Fab heavy chain are exchanged;
(ii) a second and a third antigen binding moiety that specifically bind to CEA, comprising a heavy chain variable region comprising the heavy chain CDR (HCDR) 1 of SEQ ID NO: 9, the HCDR2 of SEQ ID NO: 10, and the HCDR3 of SEQ ID NO: 11; and a light chain variable region comprising the light chain CDR (LCDR) 1 of SEQ ID NO: 12, the LCDR2 of SEQ ID NO: 13 and the LCDR3 of SEQ ID NO: 14, wherein the second and third antigen binding moiety are each a Fab molecule, particularly a conventional Fab molecule;
(iii) an Fc domain composed of a first and a second subunit,
wherein the second antigen binding moiety is fused at the C-terminus of the Fab heavy chain to the N-terminus of the Fab heavy chain of the first antigen binding moiety, and the first antigen binding moiety is fused at the C-terminus of the Fab heavy chain to the N-terminus of the first subunit of the Fc domain, and wherein the third antigen binding moiety is fused at the C-terminus of the Fab heavy chain to the N-terminus of the second subunit of the Fc domain.

In one embodiment, the first antigen binding moiety comprises a heavy chain variable region sequence that is at least about 95%, 96%, 97%, 98%, 99% or 100% identical to the amino acid sequence of SEQ ID NO: 7 and a light chain variable region sequence that is at least about 95%, 96%, 97%, 98%, 99% or 100% identical to the amino acid sequence of SEQ ID NO: 8.

In one embodiment, the first antigen binding moiety comprises the heavy chain variable region sequence of SEQ ID NO: 7 and the light chain variable region sequence of SEQ ID NO: 8.

In one embodiment, the second and third antigen binding moiety comprise a heavy chain variable region sequence that is at least about 95%, 96%, 97%, 98%, 99% or 100% identical to the amino acid sequence of SEQ ID NO: 15 and a light chain variable region sequence that is at least about 95%, 96%, 97%, 98%, 99% or 100% identical to the amino acid sequence of SEQ ID NO: 16. In one embodiment, the second and third antigen binding moieties comprise the heavy chain variable region of SEQ ID NO: 15 and the light chain variable region of SEQ ID NO: 16.

The Fc domain according to the above embodiments may incorporate, singly or in combination, all of the features described hereinabove in relation to Fc domains.

In one embodiment, the antigen binding moieties and the Fc region are fused to each other by peptide linkers, particularly by peptide linkers as in SEQ ID NO: 27 and SEQ ID NO: 28. In one embodiment, the bispecific antibody comprises a polypeptide comprising a sequence that is at least 80%, 85%, 90%, 95%, 96%, 97%, 98%, or 99% identical to the sequence of SEQ ID NO: 25, a polypeptide comprising a sequence that is at least 80%, 85%, 90%, 95%, 96%, 97%, 98%, or 99% identical to the sequence of SEQ ID NO: 26, a polypeptide comprising a sequence that is at least 80%, 85%, 90%, 95%, 96%, 97%, 98%, or 99% identical to the sequence of SEQ ID NO: 27, and a polypeptide comprising a sequence that is at least 80%, 85%, 90%, 95%, 96%, 97%, 98%, or 99% identical to the sequence of SEQ ID NO: 28.

In one embodiment, the bispecific antibody comprises a polypeptide comprising the sequence of SEQ ID NO: 25, a polypeptide comprising the sequence of SEQ ID NO: 26, a polypeptide comprising the sequence of SEQ ID NO: 27, and a polypeptide comprising the sequence of SEQ ID NO: 28. (CEA TCB)

In a particular embodiment, the CD3 antibody is CEA TCB.

In one embodiment the CD3 antibody a bispecific antibody comprising
(i) a first antigen binding moiety that specifically binds to CD3, comprising a heavy chain variable region comprising the heavy chain CDR (HCDR) 1 of SEQ ID NO: 1, the HCDR2 of SEQ ID NO: 2, and the HCDR3 of SEQ ID NO: 3; and a light chain variable region comprising the light chain CDR (LCDR) 1 of SEQ ID NO: 4, the LCDR2 of SEQ ID NO: 5 and the LCDR3 of SEQ ID NO: 6, wherein the first antigen binding moiety is a crossover Fab molecule wherein either the variable or the constant regions, particularly the variable regions, of the Fab light chain and the Fab heavy chain are exchanged;
(ii) a second and a third antigen binding moiety that specifically bind to CEA, comprising a heavy chain variable region comprising the heavy chain CDR (HCDR) 1 of SEQ ID NO: 17, the HCDR2 of SEQ ID NO: 18, and the HCDR3 of SEQ ID NO: 19; and a light chain variable region comprising the light chain CDR (LCDR) 1 of SEQ ID NO: 20, the LCDR2 of SEQ ID NO: 21 and the LCDR3 of SEQ ID NO: 22, wherein the second and third antigen binding moiety are each a Fab molecule, particularly a conventional Fab molecule;
(iii) an Fc domain composed of a first and a second subunit capable of stable association,
wherein the second antigen binding moiety is fused at the C-terminus of the Fab heavy chain to the N-terminus of the Fab heavy chain of the first antigen binding moiety, and the first antigen binding moiety is fused at the C-terminus of the Fab heavy chain to the N-terminus of the first subunit of the Fc domain, and wherein the third antigen binding moiety is fused at the C-terminus of the Fab heavy chain to the N-terminus of the second subunit of the Fc domain.

In one embodiment, the first antigen binding moiety comprises a heavy chain variable region sequence that is at least about 95%, 96%, 97%, 98%, 99% or 100% identical to the amino acid sequence of SEQ ID NO: 7 and a light chain variable region sequence that is at least about 95%, 96%, 97%, 98%, 99% or 100% identical to the amino acid sequence of SEQ ID NO: 8.

In one embodiment, the first antigen binding moiety comprises the heavy chain variable region sequence of SEQ ID NO: 7 and the light chain variable region sequence of SEQ ID NO: 8.

In one embodiment, the second and third antigen binding moiety comprise a heavy chain variable region sequence that is at least about 95%, 96%, 97%, 98%, 99% or 100% identical to the amino acid sequence of SEQ ID NO: 23 and a light chain variable region sequence that is at least about 95%, 96%, 97%, 98%, 99% or 100% identical to the amino acid sequence of SEQ ID NO: 24. In one embodiment, the second and third antigen binding moieties comprise the heavy chain variable region of SEQ ID NO: 23 and the light chain variable region of SEQ ID NO: 24.

The Fc domain according to the above embodiments may incorporate, singly or in combination, all of the features described hereinabove in relation to Fc domains.

In one embodiment, the antigen binding moieties and the Fc region are fused to each other by peptide linkers, particularly by peptide linkers as in SEQ ID NO: 30 and SEQ ID NO: 31.

In one embodiment, in the constant domain CL of the second and the third Fab molecule under (ii) the amino acid at position 124 is substituted by lysine (K) (numbering according to Kabat) and the amino acid at position 123 is substituted by lysine (K) or arginine (R), particularly by arginine (R) (numbering according to Kabat), and in the constant domain CH1 of the second and the third Fab molecule under (ii) the amino acid at position 147 is substituted by glutamic acid (E) (numbering according to Kabat EU index) and the amino acid at position 213 is substituted by glutamic acid (E) (numbering according to Kabat EU index).

In one embodiment, the bispecific antibody comprises a polypeptide comprising a sequence that is at least 80%, 85%, 90%, 95%, 96%, 97%, 98%, or 99% identical to the sequence of SEQ ID NO: 29, a polypeptide comprising a sequence that is at least 80%, 85%, 90%, 95%, 96%, 97%, 98%, or 99% identical to the sequence of SEQ ID NO: 30, a polypeptide comprising a sequence that is at least 80%, 85%, 90%, 95%, 96%, 97%, 98%, or 99% identical to the sequence of SEQ ID NO: 31, and a polypeptide comprising a sequence that is at least 80%, 85%, 90%, 95%, 96%, 97%, 98%, or 99% identical to the sequence of SEQ ID NO: 32.

In one embodiment, the bispecific antibody comprises a polypeptide comprising the sequence of SEQ ID NO: 29, a polypeptide comprising the sequence of SEQ ID NO: 30, a polypeptide comprising the sequence of SEQ ID NO: 31, and a polypeptide comprising the sequence of SEQ ID NO: 32.

For use in the treatment of cancer, the CD3 antibody would be formulated, dosed, and administered in a fashion consistent with good medical practice.
As used herein, "treatment" (and grammatical variations thereof such as "treat" or "treating") refers to clinical intervention in an attempt to alter the natural course of a disease in the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, T cell activating bispecific antigen binding molecules of the invention are used to delay development of a disease or to slow the progression of a disease. The appropriate dosage of the CD3 antibody (when used alone or in combination with one or more other additional therapeutic agents) will depend on the type of cancer to be treated, the route of administration, the body weight of the patient, the type of CD3 antibody, the severity and course of the disease, whether the CD3 antibody is administered for preventive or therapeutic purposes, previous or concurrent therapeutic interventions, the patient's clinical history and response to the CD3 antibody, and the discretion of the attending physician.
In some embodiments, the CD3 antibody is administered at a dose of about 5 mg to about 1200 mg, for example at a dose of about 5 mg, about 10 mg, about 20 mg, about 40 mg, about 60 mg, about 80 mg, about 100 mg, about 120 mg, about 150 mg, about 200 mg, about 250 mg, about 300 mg, about 400 mg, about 500 mg, about 600 mg, about 700 mg, about 800 mg, about 900 mg, about 1000 mg, about 1100 mg or about 1200 mg. In preferred embodiments, the CD3 antibody is administered at a dose of about 60 mg or more (up to about 1200 mg). In one embodiment, the CD3 antibody is administered at a dose of about 60 mg to about 600 mg. In a particular embodiment, the CD3 antibody is administered at a dose of about 60 mg. In another particular embodiment, the CD3 antibody is administered at a dose of about 80 mg. In another particular embodiment, the CD3 antibody is administered at a dose of about 100 mg. In another particular embodiment, the CD3 antibody is administered at a dose of about 160 mg. In another particular embodiment, the CD3 antibody is administered at a dose of about 400 mg. In another particular embodiment, the CD3 antibody is administered at a dose of about 600 mg. In another particular embodiment, the CD3 antibody is administered at a dose of about 800 mg. In another particular embodiment, the CD3 antibody is administered at a dose of about 1000 mg. In another particular embodiment, the CD3 antibody is administered at a dose of about 1200 mg. In some embodiments, the same dose of CD3 antibody is given at each administration. In other embodiments, the dose of the CD3 antibody is increased in subsequent administrations, as compared to the first administration. In one embodiment, the dose of the CD3 antibody is increased at each administration as compared to the previous administration, up to a dose of about 600 mg or about 1200 mg. Preferably, the dose increase is done over the course of about 3 to about 7 administrations, given once a week (QW). Increasing the dose gradually may help further reducing tumor inflammation-related adverse effects caused by the CD3 antibody in the context of the treatment described herein.

In a preferred embodiment, the CD3 antibody is administered every week / once a week (QW). In a further embodiment, the CD3 antibody is administered every three weeks (Q3W).

The present invention also relates to a (pharmaceutical) kit or package comprising a GC and/or a CD3 antibody, and instructions and/or an imprint indicating that the GC is to be employed for the treatment, amelioration and/or prophylaxis of tumor inflammation-related adverse effects caused by said CD3 antibody. Said GC and CD3 antibody are preferably packaged together in one sealed package or kit. It is also envisaged that the package or kit of the present invention, further comprises means to administer the GC and/or CD3 antibody to a patient and/or buffers, vials, teflon bags or infusion bags which are normally used for the infusion of therapeutic agents. "Means" thereby includes one or more article(s) selected from the group consisting of a syringe, a hypodermic needle, a cannula, a catheter, an infusion bag for intravenous administration, intravenous vehicles, vials, buffers, stabilizers, written instructions which aid the skilled person in the preparation of the respective doses and infusions of the invention etc.

### Examples

The following are examples of methods and compositions of the invention. It is understood that various other embodiments may be practiced, given the general description provided above.

### Example 1

### Phase I studies of the novel carcinoembryonic antigen T-cell bispecific antibody (CEA-TCB) antibody as a single agent and in combination with atezolizumab

Background: CEA-TCB (RG7802, RO6958688) is a novel T-cell bispecific antibody targeting CEA on tumor cells and CD3 on T cells. Preclinically, CEA-TCB had potent antitumor activity, leading to increased intratumoral T-cell infiltration and activation, T-cell-mediated tumor cell killing and PD-L1/PD-1 upregulation.

**Methods:** In 2 ongoing dose-escalation phase I studies, CEA-TCB is given as monotherapy IV QW (S1) or in combination (QW) with atezolizumab 1200 mg Q3W (S2) in patients with advanced CEA positive (≥ 20% of tumor cells expressing moderate or high) solid tumors. In S1, 80 patients (70 CRC) were treated at dose levels of 0.05-600 mg; in S2, 45 patients (35 CRC) at 5-160 mg. In addition biomarker analysis and PK assays were conducted.

**Results:** At doses ≥ 60 mg (31 evaluable CRC patients in S1; 14 in S2), CT scans revealed signs of tumor inflammation (lesion enlargement and perilesional edema) within 48h of the 1^{st} dose, consistent with CEA TCB mode of action. Two (6%) CRC patients in S1 (both microsatellite stable [MSS]) and 3 (21.5%) in S2 (2 MSS CRC, 1 MSI high) had confirmed partial response (PR; RECIST v1.1). Additionally, tumor reduction of -10% to -30% (stable disease) was seen in MSS CRC patients (4 [13%] in S1 and 4 [29%] in S2). At weeks 4-6, 9 (29%) CRC pts in S1 and 7 (50%) in S2 had metabolic PR (FDG PET; EORTC criteria). At all doses in S1, the most common related adverse events (AEs) were pyrexia (58%), infusion-related reactions (IRRs; 55%) and diarrhea (40%). In S1 (out of 59 patients ≥ 40 mg), the most common grade ≥3 (G3) related AEs were IRRs (24%) and diarrhea (7%). Five patients experienced dose limiting toxicities (DLTs): G3 dyspnea, G3 diarrhea, G3 hypoxia, G4 colitis and G5 respiratory failure (G4 and G5 at 600 mg [exceeding MTD (maximum tolerated dose)]). DLT events were likely associated by investigators with tumor lesion inflammation, per investigators, since they correlated with enlargement of tumor lesions and perilesional edema in the organ involved, i.e. hypoxia/dyspnea with lung lesions, colitis/diarrhea with colon or peritoneal lesions and the acute respiratory failure with tracheal obstruction induced by the enlargement of the pathological paratracheal lymph nodes. In S2, there was no evidence of new toxicities, with 2 DLTs at 160 mg (1 G3 ALT increase in a patient with liver metastases and 1 G3 maculopapular rash). Related AEs occurred mainly after doses 1 and 2.

Since the above described AEs likely related to tumor lesion inflammation, in order to mitigate or prevent tumor inflammation-related adverse effects, patients were treated with single daily doses of steroids (40 mg prednisone or 40 mg methylprednisolone, oral or i.v.) after the first administration of CEA-TCB during the first 3 days or the first 7 days depending on the dose of CEA-TCB, the number of tumor lesions, their size and their location. This prophylactic approach with steroids so far it has been useful to prevent high grade safety events (≥ grade 3) related to tumor inflammation in most patients at MTD or lower doses.

Data suggest almost linear PK in ADA negative; ADA increase CEA-TCB drug clearance and can lead to reduction or loss of exposure. In S1, at doses ≥ 60mg, the OT biopsies demonstrated a significant increase (P=0.035, 3.6 fold) in Ki67+ T cells as compared to baseline without dose-dependence (analysis ongoing for S2). In most patients, increases in IL-6 were seen after the first administration.

**Conclusions:** Evidence of antitumor activity in advanced CRC was observed during dose escalation with CEA-TCB monotherapy. Enhanced activity and a manageable safety profile was seen in combination with atezolizumab. On-treatment increases of intratumoral CD8 T cells in line with the mechanism of action support that CEA-TCB is the first tumor-targeted T cell bispecific showing consistent biological activity in a solid tumor indication.

Tumor inflammation-related adverse effects could be managed effectively by glucocorticoid administration as described herein.

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.

## Claims

1. A CD3 antibody for use in the treatment of cancer, wherein said treatment comprises administration of a glucocorticoid (GC) for the amelioration, treatment or prophylaxis of tumor inflammation-related adverse events caused by (the administration of) the CD3 antibody.

2. A method of treating cancer, comprising administration of a CD3 antibody and of a glucocorticoid (GC), wherein the GC is administered for the amelioration, treatment or prophylaxis of tumor inflammation-related adverse events caused by (the administration of) the CD3 antibody.

3. Use of a CD3 antibody in the manufacture of a medicament for the treatment of cancer, wherein said treatment comprises administration of a glucocorticoid (GC) for the amelioration, treatment or prophylaxis of tumor inflammation-related adverse events caused by (the administration of) the CD3 antibody.

4. The CD3 antibody, method or use of any one of the preceding claims, wherein the GC is methylprednisolone or prednisone.

5. The CD3 antibody, method or use of any one of the preceding claims, wherein the GC is administered at a dose of about 5 to about 60 mg, particularly about 40 mg.

6. The CD3 antibody, method or use of any one of the preceding claims, wherein the GC is administered before, concurrently with and/or after the administration of the CD3 antibody.

7. The CD3 antibody, method or use of any one of the preceding claims, wherein the GC is administered after the administration of the CD3 antibody.

8. The CD3 antibody, method or use of any one of the preceding claims, wherein the GC is administered between about 1 hour and about 7 days after the administration, particularly the first administration, of the CD3 antibody.

9. The CD3 antibody, method or use of any one of the preceding claims, wherein the GC is administered (i) at about 3 hours, about 1 day (24 hours), about 2 days (48 hours) and about 3 days (72 hours), or (ii) at about 3 hours, about 1 day (24 hours), about 2 days (48 hours), about 3 days (72 hours), about 4 days (96 hours), about 5 days (120 hours), about 6 days (144 hours) and about 7 days (168 hours) after the (first) administration of the CD3 antibody.

10. The CD3 antibody, method or use of any one of the preceding claims, wherein the cancer is a solid tumor cancer.

11. The CD3 antibody, method or use of any one of the preceding claims, wherein the cancer is a cancer selected from the group consisting of colorectal cancer, lung cancer, pancreatic cancer, breast cancer, and gastric cancer.

12. The CD3 antibody, method or use of any one of the preceding claims, wherein the CD3 antibody comprises a heavy chain variable region comprising the heavy chain CDR (HCDR) 1 of SEQ ID NO: 32, the HCDR2 of SEQ ID NO: 33, and the HCDR3 of SEQ ID NO: 34; and a light chain variable region comprising the light chain CDR (LCDR) 1 of SEQ ID NO: 35, the LCDR2 of SEQ ID NO: 36 and the LCDR3 of SEQ ID NO: 37.

13. The CD3 antibody, method or use of any one of the preceding claims, wherein the CD3 antibody is a bispecific antibody that specifically binds to CD3 and to a target cell antigen.

14. The CD3 antibody, method or use of any one of the preceding claims, wherein the CD3 antibody is a bispecific antibody that specifically binds to CD3 and to CEA.

15. The CD3 antibody, method or use of any one of the preceding claims, wherein the CD3 antibody is a bispecific antibody comprising
(i) a first antigen binding moiety that specifically binds to CD3 and comprises a heavy chain variable region comprising the heavy chain CDR (HCDR) 1 of SEQ ID NO: 32, the HCDR2 of SEQ ID NO: 33, and the HCDR3 of SEQ ID NO: 34; and a light chain variable region comprising the light chain CDR (LCDR) 1 of SEQ ID NO: 35, the LCDR2 of SEQ ID NO: 36 and the LCDR3 of SEQ ID NO: 37; and
(ii) a second antigen binding moiety that specifically binds to CEA and comprises (i) a heavy chain variable region comprising the heavy chain CDR (HCDR) 1 of SEQ ID NO: 14, the HCDR2 of SEQ ID NO: 15, and the HCDR3 of SEQ ID NO: 16; and a light chain variable region comprising the light chain CDR (LCDR) 1 of SEQ ID NO: 17, the LCDR2 of SEQ ID NO: 18 and the LCDR3 of SEQ ID NO: 19; or (ii) a heavy chain variable region comprising the heavy chain CDR (HCDR) 1 of SEQ ID NO: 136, the HCDR2 of SEQ ID NO: 137, and the HCDR3 of SEQ ID NO: 138; and a light chain variable region comprising the light chain CDR (LCDR) 1 of SEQ ID NO: 139, the LCDR2 of SEQ ID NO: 140 and the LCDR3 of SEQ ID NO: 141.

16. The CD3 antibody, method or use of any one of the preceding claims, wherein the CD3 antibody is CEA TCB.

17. The CD3 antibody, method or use of any one of the preceding claims, wherein the CD3 antibody is administered at a dose of about 60 mg to about 1200 mg.

18. The CD3 antibody, method or use of any one of the preceding claims, wherein the CD3 antibody is administered every week / once a week (QW).

19. The CD3 antibody, method or use of any one of the preceding claims, wherein the CD3 antibody is used in combination with a PD-1 axis binding antagonist.

20. The CD3 antibody, method or use of any one of the preceding claims, wherein the CD3 antibody is used in combination with atezolizumab.

21. The CD3 antibody, method or use of claim 20, wherein atezolizumab is administered at a dose of about 1200 mg every three weeks.


SEQUENCE LISTING

<110>  F. Hoffmann-La Roche AG

<120>  Treatment method

<130>  P34292

<160>  35

<170>  PatentIn version 3.5

<210>  1
<211>  5
<212>  PRT
<213>  Artificial Sequence

<220>
<223>  CD3 HCDR1

<400>  1

Thr Tyr Ala Met Asn
1               5


<210>  2
<211>  19
<212>  PRT
<213>  Artificial Sequence

<220>
<223>  CD3 HCDR2

<400>  2

Arg Ile Arg Ser Lys Tyr Asn Asn Tyr Ala Thr Tyr Tyr Ala Asp Ser
1               5                   10                  15


Val Lys Gly



<210>  3
<211>  14
<212>  PRT
<213>  Artificial Sequence

<220>
<223>  CD3 HCDR3

<400>  3

His Gly Asn Phe Gly Asn Ser Tyr Val Ser Trp Phe Ala Tyr
1               5                   10


<210>  4
<211>  14
<212>  PRT
<213>  Artificial Sequence

<220>
<223>  CD3 LCDR1

<400>  4

Gly Ser Ser Thr Gly Ala Val Thr Thr Ser Asn Tyr Ala Asn
1               5                   10


<210>  5
<211>  7
<212>  PRT
<213>  Artificial Sequence

<220>
<223>  CD3 LCDR2

<400>  5

Gly Thr Asn Lys Arg Ala Pro
1               5


<210>  6
<211>  9
<212>  PRT
<213>  Artificial Sequence

<220>
<223>  CD3 LCDR3

<400>  6

Ala Leu Trp Tyr Ser Asn Leu Trp Val
1               5


<210>  7
<211>  125
<212>  PRT
<213>  Artificial Sequence

<220>
<223>  CD3 VH

<400>  7

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Thr Tyr
            20                  25                  30


Ala Met Asn Trp Val Arg Gln Ala Pro Gly Lys Gly Leu Glu Trp Val
        35                  40                  45


Ser Arg Ile Arg Ser Lys Tyr Asn Asn Tyr Ala Thr Tyr Tyr Ala Asp
    50                  55                  60

Ser Val Lys Gly Arg Phe Thr Ile Ser Arg Asp Asp Ser Lys Asn Thr
65                  70                  75                  80

Leu Tyr Leu Gln Met Asn Ser Leu Arg Ala Glu Asp Thr Ala Val Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ser
        115                 120                 125

<210>  8
<211>  109
<212>  PRT
<213>  Artificial Sequence

<220>
<223>  CD3 VL

<400>  8

Gln Ala Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Thr Ser
            20                  25                  30

Asn Tyr Ala Asn Trp Val Gln Glu Lys Pro Gly Gln Ala Phe Arg Gly
        35                  40                  45

Leu Ile Gly Gly Thr Asn Lys Arg Ala Pro Gly Thr Pro Ala Arg Phe
    50                  55                  60

Ser Gly Ser Leu Leu Gly Gly Lys Ala Ala Leu Thr Leu Ser Gly Ala
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu
            100                 105

<210>  9
<211>  5
<212>  PRT
<213>  Artificial Sequence

<220>
<223>  CEA HCDR1

<400>  9

Glu Phe Gly Met Asn
1               5

<210>  10
<211>  17
<212>  PRT
<213>  Artificial Sequence

<220>
<223>  CEA HCDR2

<400>  10

Trp Ile Asn Thr Lys Thr Gly Glu Ala Thr Tyr Val Glu Glu Phe Lys
1               5                   10                  15

Gly

<210>  11
<211>  12
<212>  PRT
<213>  Artificial Sequence

<220>
<223>  CEA HCDR3

<400>  11

Trp Asp Phe Ala Tyr Tyr Val Glu Ala Met Asp Tyr
1               5                   10

<210>  12
<211>  11
<212>  PRT
<213>  Artificial Sequence

<220>
<223>  CEA LCDR1

<400>  12

Lys Ala Ser Ala Ala Val Gly Thr Tyr Val Ala
1               5                   10

<210>  13
<211>  7
<212>  PRT
<213>  Artificial Sequence

<220>
<223>  CEA LCDR2

<400>  13

Ser Ala Ser Tyr Arg Lys Arg
1               5

<210>  14
<211>  10
<212>  PRT
<213>  Artificial Sequence

<220>
<223>  CEA LCDR3

<400>  14

His Gln Tyr Tyr Thr Tyr Pro Leu Phe Thr
1               5                   10


<210>  15
<211>  121
<212>  PRT
<213>  Artificial Sequence

<220>
<223>  CEA VH

<400>  15

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Glu Phe
            20                  25                  30


Gly Met Asn Trp Val Arg Gln Ala Pro Gly Gln Gly Leu Glu Trp Met
        35                  40                  45


Gly Trp Ile Asn Thr Lys Thr Gly Glu Ala Thr Tyr Val Glu Glu Phe
    50                  55                  60


Lys Gly Arg Val Thr Phe Thr Thr Asp Thr Ser Thr Ser Thr Ala Tyr
65                  70                  75                  80


Met Glu Leu Arg Ser Leu Arg Ser Asp Asp Thr Ala Val Tyr Tyr Cys
                85                  90                  95


Ala Arg Trp Asp Phe Ala Tyr Tyr Val Glu Ala Met Asp Tyr Trp Gly
            100                 105                 110


Gln Gly Thr Thr Val Thr Val Ser Ser
        115                 120


<210>  16
<211>  108
<212>  PRT
<213>  Artificial Sequence

<220>
<223>  CEA VL

<400>  16

Asp Ile Gln Met Thr Gln Ser Pro Ser Ser Leu Ser Ala Ser Val Gly
1               5                   10                  15

Asp Arg Val Thr Ile Thr Cys Lys Ala Ser Ala Ala Val Gly Thr Tyr
            20                  25                  30

Val Ala Trp Tyr Gln Gln Lys Pro Gly Lys Ala Pro Lys Leu Leu Ile
        35                  40                  45

Tyr Ser Ala Ser Tyr Arg Lys Arg Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys His Gln Tyr Tyr Thr Tyr Pro Leu
                85                  90                  95

Phe Thr Phe Gly Gln Gly Thr Lys Leu Glu Ile Lys
            100                 105


<210>  17
<211>  5
<212>  PRT
<213>  Artificial Sequence

<220>
<223>  CEA HCDR1

<400>  17

Asp Thr Tyr Met His
1               5


<210>  18
<211>  17
<212>  PRT
<213>  Artificial Sequence

<220>
<223>  CEA HCDR2

<400>  18

Arg Ile Asp Pro Ala Asn Gly Asn Ser Lys Tyr Val Pro Lys Phe Gln
1               5                   10                  15

Gly

<210>  19
<211>  12
<212>  PRT
<213>  Artificial Sequence

<220>
<223>  CEA HCDR3

<400>  19

Phe Gly Tyr Tyr Val Ser Asp Tyr Ala Met Ala Tyr
1               5                   10


<210>  20
<211>  15
<212>  PRT
<213>  Artificial Sequence

<220>
<223>  CEA LCDR1

<400>  20

Arg Ala Gly Glu Ser Val Asp Ile Phe Gly Val Gly Phe Leu His
1               5                   10                  15


<210>  21
<211>  7
<212>  PRT
<213>  Artificial Sequence

<220>
<223>  CEA LCDR2

<400>  21

Arg Ala Ser Asn Arg Ala Thr
1               5


<210>  22
<211>  9
<212>  PRT
<213>  Artificial Sequence

<220>
<223>  CEA LCDR3

<400>  22

Gln Gln Thr Asn Glu Asp Pro Tyr Thr
1               5


<210>  23
<211>  121
<212>  PRT
<213>  Artificial Sequence

<220>
<223>  CEA VH

<400>  23

Gln Val Gln Leu Val Gln Ser Gly Ala Glu Val Lys Lys Pro Gly Ser
1               5                   10                  15

Ser Val Lys Val Ser Cys Lys Ala Ser Gly Phe Asn Ile Lys Asp Thr
            20                  25                  30

Tyr Met His Trp Val Arg Gln Ala Pro Gly Gln Gly Leu Glu Trp Met
        35                  40                  45

Gly Arg Ile Asp Pro Ala Asn Gly Asn Ser Lys Tyr Val Pro Lys Phe
    50                  55                  60

Gln Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Pro Phe Gly Tyr Tyr Val Ser Asp Tyr Ala Met Ala Tyr Trp Gly
            100                 105                 110

Gln Gly Thr Leu Val Thr Val Ser Ser
        115                 120


<210>  24
<211>  111
<212>  PRT
<213>  Artificial Sequence

<220>
<223>  CEA VL

<400>  24

Glu Ile Val Leu Thr Gln Ser Pro Ala Thr Leu Ser Leu Ser Pro Gly
1               5                   10                  15

Glu Arg Ala Thr Leu Ser Cys Arg Ala Gly Glu Ser Val Asp Ile Phe
            20                  25                  30

Gly Val Gly Phe Leu His Trp Tyr Gln Gln Lys Pro Gly Gln Ala Pro
        35                  40                  45

Arg Leu Leu Ile Tyr Arg Ala Ser Asn Arg Ala Thr Gly Ile Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Tyr Cys Gln Gln Thr Asn
                85                  90                  95

Glu Asp Pro Tyr Thr Phe Gly Gln Gly Thr Lys Leu Glu Ile Lys
            100                 105                 110

<210>  25
<211>  215
<212>  PRT
<213>  Artificial Sequence

<220>
<223>  CEA CD3 bsAb LC(CEA)

<400>  25

Asp Ile Gln Met Thr Gln Ser Pro Ser Ser Leu Ser Ala Ser Val Gly
1               5                   10                  15

Asp Arg Val Thr Ile Thr Cys Lys Ala Ser Ala Ala Val Gly Thr Tyr
            20                  25                  30

Val Ala Trp Tyr Gln Gln Lys Pro Gly Lys Ala Pro Lys Leu Leu Ile
        35                  40                  45

Tyr Ser Ala Ser Tyr Arg Lys Arg Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys His Gln Tyr Tyr Thr Tyr Pro Leu
                85                  90                  95

Phe Thr Phe Gly Gln Gly Thr Lys Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu

165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  26
<211>  214
<212>  PRT
<213>  Artificial Sequence

<220>
<223>  CEA CD3 bsAb LC(CD3)

<400>  26

Gln Ala Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Thr Ser
            20                  25                  30

Asn Tyr Ala Asn Trp Val Gln Glu Lys Pro Gly Gln Ala Phe Arg Gly
        35                  40                  45

Leu Ile Gly Gly Thr Asn Lys Arg Ala Pro Gly Thr Pro Ala Arg Phe
    50                  55                  60

Ser Gly Ser Leu Leu Gly Gly Lys Ala Ala Leu Thr Leu Ser Gly Ala
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu Ser Ser Ala
            100                 105                 110

Ser Thr Lys Gly Pro Ser Val Phe Pro Leu Ala Pro Ser Ser Lys Ser
        115                 120                 125

Thr Ser Gly Gly Thr Ala Ala Leu Gly Cys Leu Val Lys Asp Tyr Phe
    130                 135                 140

Pro Glu Pro Val Thr Val Ser Trp Asn Ser Gly Ala Leu Thr Ser Gly
145                 150                 155                 160

Val His Thr Phe Pro Ala Val Leu Gln Ser Ser Gly Leu Tyr Ser Leu
                165                 170                 175

Ser Ser Val Val Thr Val Pro Ser Ser Ser Leu Gly Thr Gln Thr Tyr
            180                 185                 190

Ile Cys Asn Val Asn His Lys Pro Ser Asn Thr Lys Val Asp Lys Lys
        195                 200                 205

Val Glu Pro Lys Ser Cys
    210

<210> 27
<211> 694
<212> PRT
<213> Artificial Sequence

<220>
<223> CEA CD3 bsAB HC(CEA-CD3-Fc)

<400> 27

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Glu Phe
            20                  25                  30

Gly Met Asn Trp Val Arg Gln Ala Pro Gly Gln Gly Leu Glu Trp Met
        35                  40                  45

Gly Trp Ile Asn Thr Lys Thr Gly Glu Ala Thr Tyr Val Glu Glu Phe
    50                  55                  60

Lys Gly Arg Val Thr Phe Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Trp Asp Phe Ala Tyr Tyr Val Glu Ala Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Gly Gly Gly Gly Ser Gly Gly Gly Gly Ser Glu Val Gln Leu Leu
225                 230                 235                 240

Glu Ser Gly Gly Gly Leu Val Gln Pro Gly Gly Ser Leu Arg Leu Ser
                245                 250                 255

Cys Ala Ala Ser Gly Phe Thr Phe Ser Thr Tyr Ala Met Asn Trp Val
            260                 265                 270

Arg Gln Ala Pro Gly Lys Gly Leu Glu Trp Val Ser Arg Ile Arg Ser
        275                 280                 285

Lys Tyr Asn Asn Tyr Ala Thr Tyr Tyr Ala Asp Ser Val Lys Gly Arg
    290                 295                 300

Phe Thr Ile Ser Arg Asp Asp Ser Lys Asn Thr Leu Tyr Leu Gln Met
305                 310                 315                 320

Asn Ser Leu Arg Ala Glu Asp Thr Ala Val Tyr Tyr Cys Val Arg His
                325                 330                 335

Gly Asn Phe Gly Asn Ser Tyr Val Ser Trp Phe Ala Tyr Trp Gly Gln
            340                 345                 350

Gly Thr Leu Val Thr Val Ser Ser Ala Ser Val Ala Ala Pro Ser Val
        355                 360                 365

Phe Ile Phe Pro Pro Ser Asp Glu Gln Leu Lys Ser Gly Thr Ala Ser
    370                 375                 380

Val Val Cys Leu Leu Asn Asn Phe Tyr Pro Arg Glu Ala Lys Val Gln
385                 390                 395                 400

Trp Lys Val Asp Asn Ala Leu Gln Ser Gly Asn Ser Gln Glu Ser Val
                405                 410                 415

Thr Glu Gln Asp Ser Lys Asp Ser Thr Tyr Ser Leu Ser Ser Thr Leu
            420                 425                 430

Thr Leu Ser Lys Ala Asp Tyr Glu Lys His Lys Val Tyr Ala Cys Glu
        435                 440                 445

Val Thr His Gln Gly Leu Ser Ser Pro Val Thr Lys Ser Phe Asn Arg
    450                 455                 460

Gly Glu Cys Asp Lys Thr His Thr Cys Pro Pro Cys Pro Ala Pro Glu
465                 470                 475                 480

Ala Ala Gly Gly Pro Ser Val Phe Leu Phe Pro Pro Lys Pro Lys Asp
                485                 490                 495

Thr Leu Met Ile Ser Arg Thr Pro Glu Val Thr Cys Val Val Val Asp
            500                 505                 510

Val Ser His Glu Asp Pro Glu Val Lys Phe Asn Trp Tyr Val Asp Gly
        515                 520                 525

Val Glu Val His Asn Ala Lys Thr Lys Pro Arg Glu Glu Gln Tyr Asn
    530                 535                 540

Ser Thr Tyr Arg Val Val Ser Val Leu Thr Val Leu His Gln Asp Trp
545                 550                 555                 560

Leu Asn Gly Lys Glu Tyr Lys Cys Lys Val Ser Asn Lys Ala Leu Gly
                565                 570                 575

Ala Pro Ile Glu Lys Thr Ile Ser Lys Ala Lys Gly Gln Pro Arg Glu
            580                 585                 590

Pro Gln Val Tyr Thr Leu Pro Pro Cys Arg Asp Glu Leu Thr Lys Asn
        595                 600                 605

Gln Val Ser Leu Trp Cys Leu Val Lys Gly Phe Tyr Pro Ser Asp Ile
    610                 615                 620

Ala Val Glu Trp Glu Ser Asn Gly Gln Pro Glu Asn Asn Tyr Lys Thr
625                 630                 635                 640

Thr Pro Pro Val Leu Asp Ser Asp Gly Ser Phe Phe Leu Tyr Ser Lys

                645                 650                 655

Leu Thr Val Asp Lys Ser Arg Trp Gln Gln Gly Asn Val Phe Ser Cys
            660                 665                 670

Ser Val Met His Glu Ala Leu His Asn His Tyr Thr Gln Lys Ser Leu
        675                 680                 685

Ser Leu Ser Pro Gly Lys
    690

<210> 28
<211> 451
<212> PRT
<213> Artificial Sequence

<220>
<223> CEA CD3 bsAB HC(CEA-Fc)

<400> 28

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Glu Phe
            20                  25                  30

Gly Met Asn Trp Val Arg Gln Ala Pro Gly Gln Gly Leu Glu Trp Met
        35                  40                  45

Gly Trp Ile Asn Thr Lys Thr Gly Glu Ala Thr Tyr Val Glu Glu Phe
    50                  55                  60

Lys Gly Arg Val Thr Phe Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Trp Asp Phe Ala Tyr Tyr Val Glu Ala Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
                325                 330                 335

Glu Lys Thr Ile Ser Lys Ala Lys Gly Gln Pro Arg Glu Pro Gln Val
            340                 345                 350

Cys Thr Leu Pro Pro Ser Arg Asp Glu Leu Thr Lys Asn Gln Val Ser
        355                 360                 365

Leu Ser Cys Ala Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Val Ser Lys Leu Thr Val

405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210>  29
<211>  232
<212>  PRT
<213>  Artificial Sequence

<220>
<223>  CEA CD3 bsAb LC(CD3)

<400>  29

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Ala Met Asn Trp Val Arg Gln Ala Pro Gly Lys Gly Leu Glu Trp Val
        35                  40                  45

Ser Arg Ile Arg Ser Lys Tyr Asn Asn Tyr Ala Thr Tyr Tyr Ala Asp
    50                  55                  60

Ser Val Lys Gly Arg Phe Thr Ile Ser Arg Asp Asp Ser Lys Asn Thr
65                  70                  75                  80

Leu Tyr Leu Gln Met Asn Ser Leu Arg Ala Glu Asp Thr Ala Val Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ser Ala Ser Val
        115                 120                 125

Ala Ala Pro Ser Val Phe Ile Phe Pro Pro Ser Asp Glu Gln Leu Lys
    130                 135                 140

Ser Gly Thr Ala Ser Val Val Cys Leu Leu Asn Asn Phe Tyr Pro Arg
145                 150                 155                 160

Glu Ala Lys Val Gln Trp Lys Val Asp Asn Ala Leu Gln Ser Gly Asn
                165                 170                 175

Ser Gln Glu Ser Val Thr Glu Gln Asp Ser Lys Asp Ser Thr Tyr Ser
            180                 185                 190

Leu Ser Ser Thr Leu Thr Leu Ser Lys Ala Asp Tyr Glu Lys His Lys
        195                 200                 205

Val Tyr Ala Cys Glu Val Thr His Gln Gly Leu Ser Ser Pro Val Thr
    210                 215                 220

Lys Ser Phe Asn Arg Gly Glu Cys
225                 230

<210>  30
<211>  449
<212>  PRT
<213>  Artificial Sequence

<220>
<223>  CEA CD3 bsAB HC(CEA-Fc)

<400>  30

Gln Val Gln Leu Val Gln Ser Gly Ala Glu Val Lys Lys Pro Gly Ser
1               5                   10                  15

Ser Val Lys Val Ser Cys Lys Ala Ser Gly Phe Asn Ile Lys Asp Thr
            20                  25                  30

Tyr Met His Trp Val Arg Gln Ala Pro Gly Gln Gly Leu Glu Trp Met
        35                  40                  45

Gly Arg Ile Asp Pro Ala Asn Gly Asn Ser Lys Tyr Val Pro Lys Phe
    50                  55                  60

Gln Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Pro Phe Gly Tyr Tyr Val Ser Asp Tyr Ala Met Ala Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Glu Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Glu Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
                325                 330                 335

Glu Lys Thr Ile Ser Lys Ala Lys Gly Gln Pro Arg Glu Pro Gln Val
            340                 345                 350

Cys Thr Leu Pro Pro Ser Arg Asp Glu Leu Thr Lys Asn Gln Val Ser
        355                 360                 365

Leu Ser Cys Ala Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Val Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro

<210>  31
<211>  674
<212>  PRT
<213>  Artificial Sequence

<220>
<223>  CEA CD3 bsAB HC(CEA-CD3-Fc)

<400>  31

Gln Val Gln Leu Val Gln Ser Gly Ala Glu Val Lys Lys Pro Gly Ser
1               5                   10                  15

Ser Val Lys Val Ser Cys Lys Ala Ser Gly Phe Asn Ile Lys Asp Thr
            20                  25                  30

Tyr Met His Trp Val Arg Gln Ala Pro Gly Gln Gly Leu Glu Trp Met
        35                  40                  45

Gly Arg Ile Asp Pro Ala Asn Gly Asn Ser Lys Tyr Val Pro Lys Phe
    50                  55                  60

Gln Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Pro Phe Gly Tyr Tyr Val Ser Asp Tyr Ala Met Ala Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Glu Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Glu Lys Val Glu Pro Lys Ser Cys
    210                 215                 220

Asp Gly Gly Gly Gly Ser Gly Gly Gly Gly Ser Gln Ala Val Val Thr
225                 230                 235                 240

Gln Glu Pro Ser Leu Thr Val Ser Pro Gly Gly Thr Val Thr Leu Thr
                245                 250                 255

Cys Gly Ser Ser Thr Gly Ala Val Thr Thr Ser Asn Tyr Ala Asn Trp
            260                 265                 270

Val Gln Glu Lys Pro Gly Gln Ala Phe Arg Gly Leu Ile Gly Gly Thr
        275                 280                 285

Asn Lys Arg Ala Pro Gly Thr Pro Ala Arg Phe Ser Gly Ser Leu Leu
    290                 295                 300

Gly Gly Lys Ala Ala Leu Thr Leu Ser Gly Ala Gln Pro Glu Asp Glu
305                 310                 315                 320

Ala Glu Tyr Tyr Cys Ala Leu Trp Tyr Ser Asn Leu Trp Val Phe Gly
                325                 330                 335

Gly Gly Thr Lys Leu Thr Val Leu Ser Ser Ala Ser Thr Lys Gly Pro
            340                 345                 350

Ser Val Phe Pro Leu Ala Pro Ser Ser Lys Ser Thr Ser Gly Gly Thr
        355                 360                 365

Ala Ala Leu Gly Cys Leu Val Lys Asp Tyr Phe Pro Glu Pro Val Thr
    370                 375                 380

Val Ser Trp Asn Ser Gly Ala Leu Thr Ser Gly Val His Thr Phe Pro
385                 390                 395                 400

Ala Val Leu Gln Ser Ser Gly Leu Tyr Ser Leu Ser Ser Val Val Thr
                405                 410                 415

Val Pro Ser Ser Ser Leu Gly Thr Gln Thr Tyr Ile Cys Asn Val Asn
            420                 425                 430

His Lys Pro Ser Asn Thr Lys Val Asp Lys Lys Val Glu Pro Lys Ser
        435                 440                 445

Cys Asp Lys Thr His Thr Cys Pro Pro Cys Pro Ala Pro Glu Ala Ala
    450                 455                 460

Gly Gly Pro Ser Val Phe Leu Phe Pro Pro Lys Pro Lys Asp Thr Leu
465                 470                 475                 480

Met Ile Ser Arg Thr Pro Glu Val Thr Cys Val Val Val Asp Val Ser
                485                 490                 495

His Glu Asp Pro Glu Val Lys Phe Asn Trp Tyr Val Asp Gly Val Glu
            500                 505                 510

Val His Asn Ala Lys Thr Lys Pro Arg Glu Glu Gln Tyr Asn Ser Thr
        515                 520                 525

Tyr Arg Val Val Ser Val Leu Thr Val Leu His Gln Asp Trp Leu Asn
    530                 535                 540

Gly Lys Glu Tyr Lys Cys Lys Val Ser Asn Lys Ala Leu Gly Ala Pro
545                 550                 555                 560

Ile Glu Lys Thr Ile Ser Lys Ala Lys Gly Gln Pro Arg Glu Pro Gln
                565                 570                 575

Val Tyr Thr Leu Pro Pro Cys Arg Asp Glu Leu Thr Lys Asn Gln Val
            580                 585                 590

Ser Leu Trp Cys Leu Val Lys Gly Phe Tyr Pro Ser Asp Ile Ala Val
        595                 600                 605

Glu Trp Glu Ser Asn Gly Gln Pro Glu Asn Asn Tyr Lys Thr Thr Pro
    610                 615                 620

Pro Val Leu Asp Ser Asp Gly Ser Phe Phe Leu Tyr Ser Lys Leu Thr

625                 630                 635                 640

Val Asp Lys Ser Arg Trp Gln Gln Gly Asn Val Phe Ser Cys Ser Val
                645                 650                 655

Met His Glu Ala Leu His Asn His Tyr Thr Gln Lys Ser Leu Ser Leu
            660                 665                 670

Ser Pro

<210>  32
<211>  218
<212>  PRT
<213>  Artificial Sequence

<220>
<223>  CEA CD3 bsAb LC(CEA)

<400>  32

Glu Ile Val Leu Thr Gln Ser Pro Ala Thr Leu Ser Leu Ser Pro Gly
1               5                   10                  15

Glu Arg Ala Thr Leu Ser Cys Arg Ala Gly Glu Ser Val Asp Ile Phe
            20                  25                  30

Gly Val Gly Phe Leu His Trp Tyr Gln Gln Lys Pro Gly Gln Ala Pro
        35                  40                  45

Arg Leu Leu Ile Tyr Arg Ala Ser Asn Arg Ala Thr Gly Ile Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Tyr Cys Gln Gln Thr Asn
                85                  90                  95

Glu Asp Pro Tyr Thr Phe Gly Gln Gly Thr Lys Leu Glu Ile Lys Arg
            100                 105                 110

Thr Val Ala Ala Pro Ser Val Phe Ile Phe Pro Pro Ser Asp Arg Lys
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  33
<211>  225
<212>  PRT
<213>  Homo sapiens

<400>  33

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro
225

<210>  34
<211>  207
<212>  PRT
<213>  Homo sapiens

<400>  34

Met Gln Ser Gly Thr His Trp Arg Val Leu Gly Leu Cys Leu Leu Ser
1               5                   10                  15

Val Gly Val Trp Gly Gln Asp Gly Asn Glu Glu Met Gly Gly Ile Thr
            20                  25                  30

Gln Thr Pro Tyr Lys Val Ser Ile Ser Gly Thr Thr Val Ile Leu Thr
        35                  40                  45

Cys Pro Gln Tyr Pro Gly Ser Glu Ile Leu Trp Gln His Asn Asp Lys
    50                  55                  60

Asn Ile Gly Gly Asp Glu Asp Asp Lys Asn Ile Gly Ser Asp Glu Asp
65                  70                  75                  80

His Leu Ser Leu Lys Glu Phe Ser Glu Leu Glu Gln Ser Gly Tyr Tyr
                85                  90                  95

Val Cys Tyr Pro Arg Gly Ser Lys Pro Glu Asp Ala Asn Phe Tyr Leu
            100                 105                 110

Tyr Leu Arg Ala Arg Val Cys Glu Asn Cys Met Glu Met Asp Val Met
        115                 120                 125

Ser Val Ala Thr Ile Val Ile Val Asp Ile Cys Ile Thr Gly Gly Leu
    130                 135                 140

Leu Leu Leu Val Tyr Tyr Trp Ser Lys Asn Arg Lys Ala Lys Ala Lys
145                 150                 155                 160

Pro Val Thr Arg Gly Ala Gly Ala Gly Gly Arg Gln Arg Gly Gln Asn
                165                 170                 175

Lys Glu Arg Pro Pro Pro Val Pro Asn Pro Asp Tyr Glu Pro Ile Arg
            180                 185                 190

Lys Gly Gln Arg Asp Leu Tyr Ser Gly Leu Asn Gln Arg Arg Ile
        195                 200                 205

<210>  35
<211>  198
<212>  PRT
<213>  Macaca fascicularis

<400>  35

Met Gln Ser Gly Thr Arg Trp Arg Val Leu Gly Leu Cys Leu Leu Ser
1               5                   10                  15

Ile Gly Val Trp Gly Gln Asp Gly Asn Glu Glu Met Gly Ser Ile Thr
            20                  25                  30

Gln Thr Pro Tyr Gln Val Ser Ile Ser Gly Thr Thr Val Ile Leu Thr
        35                  40                  45

Cys Ser Gln His Leu Gly Ser Glu Ala Gln Trp Gln His Asn Gly Lys
    50                  55                  60

Asn Lys Glu Asp Ser Gly Asp Arg Leu Phe Leu Pro Glu Phe Ser Glu
65                  70                  75                  80

Met Glu Gln Ser Gly Tyr Tyr Val Cys Tyr Pro Arg Gly Ser Asn Pro
                85                  90                  95

Glu Asp Ala Ser His His Leu Tyr Leu Lys Ala Arg Val Cys Glu Asn
            100                 105                 110

Cys Met Glu Met Asp Val Met Ala Val Ala Thr Ile Val Ile Val Asp
        115                 120                 125

Ile Cys Ile Thr Leu Gly Leu Leu Leu Leu Val Tyr Tyr Trp Ser Lys
    130                 135                 140

Asn Arg Lys Ala Lys Ala Lys Pro Val Thr Arg Gly Ala Gly Ala Gly
145                 150                 155                 160

Gly Arg Gln Arg Gly Gln Asn Lys Glu Arg Pro Pro Pro Val Pro Asn
                165                 170                 175

Pro Asp Tyr Glu Pro Ile Arg Lys Gly Gln Gln Asp Leu Tyr Ser Gly
            180                 185                 190

Leu Asn Gln Arg Arg Ile
        195